# EUROPEAN PATENT APPLICATION

(11) **EP 4 687 255 A1**
(43) Date of publication of application: **04.02.2026**
(21) Application number: 24200107.1
(22) Date of filing: 12.09.2024
(51) Int. Cl.: H02J 7/00, A45D 27/46, A61L 2/10, A61L 2/26, B26B 19/38, B26B 19/14

(54) **AUXILIARY DEVICE FOR STORING, ELECTRICALLY CHARGING AND DISINFECTING A PERSONAL CARE DEVICE**

(30) Priority: 29.07.2024 WO PCT/CN2024/108227
(71) Applicant: Koninklijke Philips N.V., 5656 AG Eindhoven (NL)
(72) Inventor: WANG, Menglu, 5656 AG Eindhoven (NL); JANSEN, Johannes Antonius, 5656 AG Eindhoven (NL); GÖZÜTOK, Ahmet, 5656 AG Eindhoven (NL); ZHANG, Haixing, 5656 AG Eindhoven (NL); YANG, Jing, 5656 AG Eindhoven (NL); ZHANG, Chengye, 5656 AG Eindhoven (NL)
(74) Representative: Philips Intellectual Property & Standards

(57) **Abstract**

The invention relates to an auxiliary device (5) for disinfecting, electrically charging and storing a personal care device (3) such as an electric shaver. The auxiliary device has a lower housing portion (25) for receiving a first portion (55) of a main body (7) of the personal care device and an upper housing portion (27) for receiving a second portion (65) of the main body and a personal care unit (9) of the personal care device which is coupled to the main body. In a normal operational position of the auxiliary device, a longitudinal axis (41, 49) of the lower and upper housing portions extends in vertical direction, so that the personal care device is received in a vertical standing orientation within the auxiliary device. The lower housing portion has an electric charging contact (63) for engaging an electric charging contact (19) of the main body. The upper housing portion has a light source (73a, 73b, 73c) for exposing the personal care unit to disinfecting light in a closed condition of the upper housing portion. In an opened condition of the upper housing portion, the second portion of the main body protrudes from the lower housing portion and can be easily grasped by the user when placing and removing the personal care device into and from the auxiliary device. The lower and upper housing portions fully enclose the personal care device in the closed condition of the upper housing portion.

## Description

### FIELD OF THE INVENTION

The invention relates to an auxiliary device for use with an electric personal care device and configured to store the personal care device, to electrically charge a battery of the personal care device, and to disinfect a personal care unit of the personal care device by means of disinfecting light, wherein the personal care device comprises a main body accommodating the battery and having a longitudinal axis which extends from a first end portion of the main body to a second end portion of the main body, wherein the first end portion is provided with an electric charging contact for charging the battery, and wherein the personal care unit is coupled to the second end portion.

The invention also relates to a personal care system comprising an electric personal care device and an auxiliary device which is configured to store the personal care device, to electrically charge a battery of the personal care device, and to disinfect a personal care unit of the personal care device by means of disinfecting light, wherein the personal care device comprises a main body accommodating the battery and having a longitudinal axis which extends from a first end portion of the main body to a second end portion of the main body, wherein the first end portion is provided with an electric charging contact for charging the battery, and wherein the personal care unit is coupled to the second end portion.

### BACKGROUND OF THE INVENTION

Personal care devices configured to provide a personal care treatment to a body part of a subject are widely known. Examples are electric shavers to shave beard hairs on the face of a human, hair-trimming devices to cut beard hairs on the face of a human or hairs on human body parts other than the face, intense pulsed light (IPL) devices to remove hairs on the legs or other body parts of a human, facial-cleansing brushing devices to clean the face of a human by means of a rotating and/or vibrating brush, and skin rejuvenation devices to rejuvenate the skin of body parts of a human by means of electromagnetic or radiofrequency energy. Such personal care devices usually have a main body and a personal care unit that is coupled to the main body and actually provides the personal care treatment to the body part. The personal care unit may be fixedly coupled to the main body or releasably coupled to the main body, so that different personal care units may be selectively coupled to the main body by the user to enable different personal care treatments. After one or more treatment sessions with the personal care unit of the personal care device, the personal care unit may need to be cleaned and/or disinfected. In this respect, it is known to use a personal care device with a disinfecting device that is configured to generate ultraviolet, UV, disinfecting light, or another type of disinfecting light, to disinfect the personal care unit. UV light is known for its strong disinfecting properties and is effective in inactivating microorganisms by damaging their DNA and RNA. Disinfection of the personal unit unit will reduce the risk of infection and inflammation of the body part to be treated by microorganisms present on parts of the personal care unit which are in contact with the body part to be treated.

The main body of known personal care devices may accommodate a battery, in particular a rechargeable battery. The battery may power, e.g., an electric motor, which may be arranged in the main body to drive a movable treatment component of the personal care unit when coupled to the main body. The battery may also power other electric components of the personal care device. In the example of an electric shaver, the motor may drive the moveable cutting members of the hair-cutting units of a shaving unit of the electric shaver when coupled to the main body. It is also known to provide a disinfecting device, configured to disinfect the personal care unit of the personal care device by means of disinfecting light as described here before, with an additional charging function to electrically charge the battery of the personal care device when placed in the disinfecting device. For this purpose, the main body of the personal care device may be provided with an electric charging contact at a first end portion of the main body. The personal care unit may be coupled to a second end portion of the main body, and the main body may have a longitudinal axis which extends from the first end portion to the second end portion. Such a combined disinfecting and charging device may have a light source for generating the disinfecting light arranged to expose the personal care unit with the disinfecting light, and may further have an electric charging contact arranged to be electrically connected to the electric charging contact of the main body, when the personal care device is placed in the combined disinfecting and charging device. It is further known that such a combined disinfecting and charging device may have an additional storing function to store the personal care device when the personal care device is not in use.

KR20200111078A discloses a charging device for an electric shaver which has an additional disinfecting function. The charging device comprises a UV light module arranged to irradiate the shaving unit of the shaver with UV light when the shaver is placed in the charging device for being charged. The charging device comprises a base portion having a receiving space configured to receive a lower part of the main body of the shaver such as to hold the shaver in an upright position, as is commonly known in the area of charging devices for electric shavers or other personal care devices, such as electric toothbrushes. The base portion is provided with electric charging contacts which are connectable with electric charging contacts provided on the lower part of the main body of the shaver when the shaver is placed in the base portion. The UV light module is arranged in an upper housing portion of the charging device, which is connected to the base portion via a vertically extending column and positioned vertically above the base portion. When the shaver is placed in the base portion, a main portion of the main body and the shaving unit of the shaver are present in an open area between the base portion and the upper housing portion, wherein the shaving unit is in a position close to the upper housing portion of the charging device so that, during charging of the shaver, the shaving unit can be irradiated and disinfected by the UV light emitted by the UV light module. A disadvantage of this known charging device is that, because the shaving unit is present in said open area between the base portion and the upper housing portion of the charging device, the user may be exposed to the UV light, e.g., UV light emitted directly by the UV light module or UV light reflected by parts of the shaving unit. This imposes a serious risk of, e.g., damage to the user's eyes. Another disadvantage of this known charging device is that placement and removal of the shaver by the user into and from the base portion of the charging device may be difficult as a result of the limited space between the shaving unit and the upper housing portion and the fixed position of the upper housing portion relative to the base portion. Another disadvantage of this known charging device is that its suitability to store the shaver for a longer time is limited, in particular when the charging device and the shaver stored therein are to be transported during, e.g., travelling of the user.

US 2005/0189907 A1 discloses a combined disinfecting and charging device configured to disinfect the shaving unit of an electric shaver by means of disinfecting light and to charge a battery of the shaver. The known disinfecting and charging device comprises a base member having a longitudinal receiving groove formed on the upper surface of the base member. The receiving groove is configured to stably receive and mount the shaver, including the main body and the shaving unit of the shaver, in a position which is slightly inclined relative to a horizontal position. When the shaver is received by the receiving groove, a battery terminal, provided on an end portion of the main body of the shaver remote from the shaving unit, is in electrical contact with a charging terminal provided on the base member of the disinfecting and charging device. This allows the battery in the main body of the shaver to be charged when the shaver is placed in the disinfecting and charging device. When the shaver is received by the receiving groove, the shaving unit of the shaver is in a position adjacent to a UV lamp, which is also arranged on the upper surface of the base member of the disinfecting and charging device. This allows the shaving unit to be disinfected by the UV light generated by the UV lamp. The disinfecting and charging device further comprises a cover member which is hinged onto the base member. By closing the cover member, the receiving groove accommodating the shaver is fully covered. An on/off switch to activate the UV lamp is also arranged on the upper surface of the base member and is turned on by contact with the cover member when the cover member is pivoted into the closed position. A disadvantage of this known disinfecting and charging device is that it occupies a relatively large surface area, in particular when placed on a horizontal supporting surface. This may be inconvenient and impractical in an environment with limited space, such as in a bathroom. Another disadvantage of this known disinfecting and charging device is that at least the base member of the device is rather bulky. As a result, it is not convenient for the user to use the device, together with the shaver, as a so-called "on-the-go" product suitable for being transported during travelling of the user. Another disadvantage of this known disinfecting and charging device is that placement of the shaver by the user into the base member and, in particular, removal of the shaver from the base member of the device may be difficult, because the receiving groove in the base member is configured to receive the shaver over its full longitudinal extension, including the shaving unit and the end portion of the main body of the shaver remote from the shaving unit. To facilitate placement and removal of the shaver to some extent, the base member of the known disinfecting and charging device is provided with grasping recesses, but such grasping recesses do not support an intuitive handling of the shaver by the user and also complicate the layout of the base member.

### SUMMARY OF THE INVENTION

It is an object of the present invention to provide an auxiliary device, for use with an electric personal care device, of a type as described in the section "field of the invention" which does not have the disadvantages of the known disinfecting and charging devices as described in the section "background of the invention". In particular, an object of the present invention is to provide an auxiliary device, for use with an electric personal care device, as described in the section "field of the invention" which occupies only a relatively small surface area in particular when placed on a horizontal supporting surface, which enables placement and removal of the personal care device by the user into and from the auxiliary device in a convenient and intuitive manner, which is safe to use in particular by preventing the disinfecting light from reaching the user's eyes, and which is suitable for storing the personal care device in a safe and convenient manner in particular when the auxiliary device and the personal care device stored therein are to be transported during, e.g., travelling of the user.

To achieve the above-mentioned objects, the present invention provides an auxiliary device for use with an electric personal care device and configured to store the personal care device, to electrically charge a battery of the personal care device, and to disinfect a personal care unit of the personal care device by means of disinfecting light, wherein:
- the personal care device comprises a main body accommodating the battery and having a longitudinal axis which extends from a first end portion of the main body to a second end portion of the main body;
- the first end portion is provided with an electric charging contact for charging the battery;
- the personal care unit is coupled to the second end portion;
- the main body has a first portion which includes the first end portion, extends along the longitudinal axis from the first end portion towards the second end portion, and excludes a second portion of the main body, said second portion including the second end portion and extending along the longitudinal axis from the second end portion towards the first end portion; and
- the main body and the second portion of the main body have, respectively, a first average length and a second average length measured parallel to the longitudinal axis;
   wherein the auxiliary device comprises a housing having a lower housing portion and an upper housing portion, wherein the lower housing portion comprises:
   - a bottom side configured to be supportable in a horizontal orientation which defines a normal operational position of the auxiliary device;
   - an upper side provided with a receiving opening;
   - a longitudinal axis extending, in the normal operational position, in a vertical direction from the bottom side to the upper side; and
   - a first receiving chamber which is accessible via the receiving opening and configured to receive the first portion of the main body in a predefined stored position of the personal care device; wherein the upper housing portion:
   - has a closed condition and an opened condition relative to the lower housing portion; and
   - comprises a second receiving chamber which is configured to receive, in the closed condition of the upper housing portion and in the predefined stored position of the personal care device, the second portion of the main body and the personal care unit coupled to the main body;
      and wherein:
      - the lower housing portion and the upper housing portion are configured to fully enclose the main body and the personal care unit coupled to the main body in the closed condition of the upper housing portion and in the predefined stored position of the personal care device;
      - the lower housing portion is configured such that, in the opened condition of the upper housing portion and in the predefined stored position of the personal care device, the second portion of the main body protrudes from the lower housing portion via the receiving opening;
      - the lower housing portion comprises an electric charging contact arranged to engage the electric charging contact of the main body in the predefined stored position of the personal care device;
      - the upper housing portion comprises at least one light source configured to generate the disinfecting light and arranged to expose the personal care unit, when coupled to the main body, with the disinfecting light in the closed condition of the upper housing portion and in the predefined stored position of the personal care device; and
      - a ratio between the second average length and the first average length is at least 0.05.

The auxiliary device according to the invention comprises a lower housing portion and an upper housing portion, wherein the upper housing portion has a closed condition and an opened condition relative to the lower housing portion. In the opened condition of the upper housing portion, a first receiving chamber in the lower housing portion is accessible via a receiving opening which is provided at an upper side of the lower housing portion. The first receiving chamber is configured to receive a first portion of a main body of a personal care device in a predefined stored position. The first portion of the main body includes a first end portion of the main body, which is provided with an electric charging contact for charging a battery accommodated in the main body. In the opened condition of the upper housing portion and in the predefined stored position of the personal care device, a second portion of the main body, to which a personal care unit of the personal care device is coupled, protrudes from the lower housing portion via the receiving opening. In a normal operational position of the auxiliary device, a bottom side of the lower housing portion is supported in a horizontal orientation, wherein a longitudinal axis of the lower housing portion extends in a vertical direction from the bottom side to the upper side of the lower housing portion. Thus, the lower housing portion is configured to receive the first portion of the main body in a substantial vertical orientation, i.e., in an orientation wherein a longitudinal axis of the main body of the personal care device extends in a substantial vertical direction. This allows the lower housing portion to have dimensions in directions perpendicular to its longitudinal axis which are small relative to an axial dimension of the housing of the auxiliary device measured parallel to the longitudinal axis of the lower housing portion. In particular, the bottom side of the lower housing portion, by which the auxiliary device is to be supported by a horizontal external supporting surface, e.g., a horizontal table surface, may have relatively small dimensions, so that the auxiliary device may occupy only a relatively small surface area when placed on said horizontal supporting surface.

Furthermore, as a result of the substantially vertical orientation of the lower housing portion in the normal operational position of the auxiliary device, wherein the receiving opening is present at the upper side of the lower housing portion, a user may, in the opened condition of the upper housing portion, conveniently place the main body of the personal care device into the first receiving chamber via said receiving opening, and may also conveniently remove the main body from the first receiving chamber via said receiving opening. Because, in the predefined stored position of the personal care device, the second portion of the main body, to which the personal care unit is coupled, protrudes from the lower housing portion via the receiving opening, a user may conveniently and intuitively grasp the second portion of the main body at two mutually opposite locations on its circumference during placing and removing the main body into and from the lower housing portion. For this purpose, in accordance with the invention, a ratio between a second average length of the second portion of the main body and a first average length of the main body in total, both measured parallel to the longitudinal axis of the main body, is at least 0.05. In general, for most commonly used personal care devices having a main body with an elongated shape, this ratio allows the user sufficient space to grasp the second portion of the main body, when protruding from the lower housing portion, by means of at least two fingers without a need to touch the personal care unit coupled to the second end portion of the main body. Because the user does not need to touch the personal care unit during placing and removing the personal care device into and from the lower housing portion, it is prevented that the personal care unit may unintentionally be decoupled from the main body during said placing and removing, in particular in embodiments wherein the personal care unit is releasably coupled to the main body in order to be exchangeable by the user. Finally, because the upper housing portion has an opened condition relative to the lower housing portion, the upper housing portion may not hinder the user during said placing and removing of the personal care device.

In the closed condition of the upper housing portion, and with the personal care device being placed in the predefined stored position in the first receiving chamber of the lower housing portion, the second portion of the main body of the personal care device and the personal care unit coupled to the main body are received by a second receiving chamber which is provided in the upper housing portion. In said closed condition of the upper housing portion, and with the personal care device being places in said predefined stored position, at least one light source arranged in the upper housing portion may generate the disinfecting light and expose the personal care unit with the disinfecting light to disinfect the personal care unit. Furthermore, in said predefined stored position of the personal care device, an electric charging contact arranged in the lower housing portion engages the electric charging contact of the main body of the personal care device, so that the battery of the personal care device may be electrically charged, in particular also in the closed condition of the upper housing portion.

Furthermore, in the closed condition of the upper housing portion, with the personal care device being placed in the predefined stored position, the lower housing portion and the upper housing portion fully enclose the main body of the personal care device and the personal care unit coupled to the main body. In other words, in said closed condition the first receiving chamber of the lower housing portion, which receives the first portion of the main body, and the second receiving chamber of the upper housing portion, which receives the second portion of the main body and the personal care unit coupled to the main body, jointly form a fully closed cavity accommodating the personal care device in total. This will prevent, in the closed condition of the upper housing portion, leakage of the disinfecting light, generated by the light source in the upper housing portion, to the environment of the auxiliary device and, thus, will prevent any safety risks for the user, in particular for the user's eyes. In addition, said fully closed cavity, formed by the first and second receiving chambers in the closed condition of the upper housing portion, will protect the personal care device, when being stored for a longer time in the auxiliary device, against any external loads or other types of external impacts. This enables, e.g., safe and convenient transportation of the auxiliary device and the personal care device stored therein during, e.g., travelling of the user.

In a preferred embodiment of the auxiliary device according to the invention, the ratio between the second average length of the second portion of the main body of the personal are device and the first average length of the main body in total is from 0.1 to 0.35. Said preferred ratio allows the user to conveniently and intuitively grasp the second portion of the main body, when protruding from the lower housing portion in the opened condition of the upper housing portion, without a need to touch the personal care unit coupled to the second end portion of the main body. Said preferred ratio also enables, in the opened condition of the upper housing portion, a stable support of the first portion of the main body of the personal care device within the first receiving chamber of the lower housing portion after placement of the personal care device into the predefined stored position, so that the upper housing portion may be moved from the opened condition into the closed condition without being obstructed by an incorrect position of the personal care device in the lower housing portion.

In a preferred embodiment of the auxiliary device according to the invention, the upper housing portion is pivotable relative to the lower housing portion from the closed condition to the opened condition, and vice versa, by means of a hinge structure. The hinge structure allows the user to conveniently move the upper housing portion from the closed condition to the opened condition, and vice versa. According to the invention, however, the closed and opened conditions of the upper housing portion relative to the lower housing portion may be realized in alternative ways. E.g., the upper housing portion may be releasably coupled to the lower housing portion and may, e.g., be decoupled and fully removed from the lower housing portion by the user.

In a particular embodiment of the auxiliary device according to the invention, wherein the upper housing portion is pivotable relative to the lower housing portion by means of a hinge structure, a main orientation of the receiving opening of the lower housing portion is oblique relative to the longitudinal axis of the lower housing portion, the lower housing portion has an edge structure surrounding the receiving opening, and the hinge structure is provided in a position on the edge structure where the edge structure has a largest distance from the bottom side of the lower housing portion, measured in a direction parallel to the longitudinal axis of the lower housing portion. Said oblique orientation of the receiving opening, combined with an arrangement of the hinge structure in an uppermost position on the edge structure relative to the bottom side of the lower housing portion, enables the user to even more conveniently place and remove the personal care device into and from the lower housing portion in the opened condition of the upper housing portion. Furthermore, said arrangement of the hinge structure in an uppermost position on the edge structure relative to the bottom side of the lower housing portion minimizes the risk for the upper housing portion of being obstructed by the personal care device, in its predefined stored position in the lower housing portion, when pivoting relative to the lower housing portion.

In a further embodiment of the auxiliary device according to the invention, an upper side of the main body of the personal care device, which is provided at the second end portion of the main body, has a main orientation which is oblique relative to the longitudinal axis of the main body, and a main orientation of the receiving opening of the lower housing portion is parallel to the main orientation of the upper side of the main body in the predefined stored position of the personal care device. As a result of the main orientation of the receiving opening being parallel to the main orientation of the upper side of the main body in the predefined stored position of the personal care device, a distance over which the second portion of the main body protrudes from the lower housing portion in the opened condition of the upper housing portion may be substantially identical along a circumference of the second portion. This will further improve the convenience for the user of grasping the second portion of main body during placing and removing the personal care device into and from the lower housing portion.

In a preferred embodiment of the auxiliary device according to the invention, the electric charging contact of the personal care device is provided on a lower side of the main body provided at the first end portion of the main body, the electric charging contact of the auxiliary device is provided on a bottom surface of the first receiving chamber, and the first receiving chamber tapers in a direction along the longitudinal axis of the lower housing portion from the receiving opening towards the bottom surface. The arrangement of the electric charging contact of the personal care device on the lower side of the main body and the arrangement of the electric charging contact of the auxiliary device on the bottom surface of the first receiving chamber enable a solid and secure engagement of the electric charging contact of the personal care device with the electric charging contact of the auxiliary device under the influence of gravity force during and after placement of the personal care device into the lower housing portion. Said tapering of the first receiving chamber enables the electric charging contact of the personal care device to be correctly aligned for engagement with the electric charging contact of the auxiliary device during placement of the personal care device into the lower housing portion.

In a particular embodiment of the auxiliary device according to the invention, the personal care device is an electric shaver and the personal care unit is a shaving unit comprising three circularly shaped hair-cutting units which are arranged triangularly on a supporting member of the shaving unit, and the upper housing portion of the auxiliary device comprises three light sources, in particular three UV-LEDs, arranged triangularly to each mainly expose a respective one of the three hair-cutting units of the shaving unit, when coupled to the main body, with the disinfecting light in the closed condition of the upper housing portion and in the predefined stored position of the electric shaver. During use of an electric shaver having a shaving unit with three triangularly arranged circular hair-cutting units, which type of electric shaver is very well known to the skilled person, the user's skin is mainly in contact with the three hair-cutting units. Therefore, in particular said hair-cutting units should be exposed to the disinfecting light during a disinfecting process by means of the auxiliary device. In this particular embodiment of the auxiliary device according to the invention, the triangular arrangement of the three light sources in the upper housing portion of the auxiliary device enables an effective and efficient exposure of the three hair-cutting units of the shaving unit to the disinfecting light. It is noted that, for an effective and efficient exposure of the shaving unit of a different type of electric shaver or, more generally, the personal care unit of a personal care device of a type different from an electric shaver, the number of light sources provided in the upper housing portion and the mutual arrangement of the light sources in the upper housing portion may be different from the number and the mutual arrangement of the light sources in this particular embodiment of the auxiliary device.

In a further embodiment of the auxiliary device according to the invention, one of the lower housing portion and the upper housing portion comprises a first magnet, and the other one of the lower housing portion and the upper housing portion comprises a first magnetic-field sensor, in particular a first Hall sensor, wherein the first magnet and the first magnetic-field sensor are arranged to enable the first magnetic-field sensor to detect different magnetic-field strengths in, respectively, the closed condition and the opened condition of the upper housing portion, and wherein the auxiliary device comprises a processor configured to activate the at least one light source for a predetermined time period upon detection of both the closed condition of the upper housing portion by means of the first magnetic-field sensor and the predefined stored position of the personal care device by means of an electric detection signal exchanged via the electric charging contacts of the auxiliary device and the personal care device when mutually engaging. In this further embodiment, the first magnetic-field sensor may detect the closed condition of the upper housing portion because, in said closed condition, the first magnet has a predetermined position relative to the first magnetic-field sensor corresponding to a predetermined magnetic-field strength present at the first magnetic-field sensor. Thus, the processor may automatically start the disinfecting process, by activation of the light source, upon detection of the closed condition of the upper housing portion by means of the first magnetic-field sensor. Because the processor is also enabled, by means of said electric detection signal exchanged via the electric charging contacts of the auxiliary device and the personal care device when mutually engaging, whether or not the personal care device is in the predefined stored position, the processor may automatically activate the light source only when, in addition to the closed condition of the upper housing portion, also the presence of the personal care device in the auxiliary device is detected. Thereby, it is prevented that the light source will be activated, upon closing of the auxiliary device, when the personal care device is not present in the auxiliary device. The processor may automatically deactivate the light source after said predetermined time period.

In a yet further embodiment of the auxiliary device according to the invention, the processor is configured to deactivate the at least one light source and/or disable activation of the at least one light source when the first magnetic-field sensor does not detect the closed condition of the upper housing portion. Thereby, the processor may deactivate the light source when, during a disinfecting process with the upper housing portion in its closed condition, the user, e.g., unintentionally opens the auxiliary device by moving the upper housing portion into its opened condition. The processor may also prevent activation of the light source when and/or as long as the upper housing portion is not in its closed condition. In this way, leakage of the disinfecting light to the environment of the auxiliary device and, thus, safety risks for the user, in particular the user's eyes, are prevented when the upper housing portion is not in its closed condition.

In a yet further embodiment of the auxiliary device according to the invention, the processor is configured to activate the at least one light source by closing a first electric switch connected in series with the at least one light source, said one of the lower housing portion and the upper housing portion comprises a second magnet and said other one of the lower housing portion and the upper housing portion comprises a second magnetic-field sensor, in particular a second Hall sensor, the second magnet and the second magnetic-field sensor are arranged to enable the second magnetic-field sensor to detect different magnetic-field strengths in, respectively, the closed condition and the opened condition of the upper housing portion, and the auxiliary device comprises a second electric switch connected in series with the first electric switch and the at least one light source and configured to be opened as a result of an output signal provided by the second magnetic-field sensor when the second magnetic-field sensor does not detect the closed condition of the upper housing portion. In this yet further embodiment, the processor may automatically activate the light source, by closing said first electric switch, upon detection of the closed condition of the upper housing portion by the first magnetic-field sensor. The processor may also automatically deactivate the light source, as a safety measure and by opening said first electric switch, when during a disinfecting process the first magnetic-field sensor no longer detects said closed condition. In this yet further embodiment, the second magnetic-field sensor may detect, by cooperation with the second magnet, the closed condition of the upper housing portion in a way similar to the first magnetic-field sensor by its cooperation with the first magnet as described herein before. The second magnetic-field sensor is however arranged to directly control, via its output signal, the second electric switch connected in series with the first electric switch. In particular, as an additional safety measure, the second electric switch is opened and, thereby, the light source is deactivated by the output signal of the second magnetic-field sensor when the second magnetic-field sensor does not detect the closed condition of the upper housing portion. Because the second electric switch is connected in series with the first electric switch and the light source, the first and second electric switches should both be closed in order to activate the light source.

In a yet further embodiment of the auxiliary device according to the invention, said other one of the lower housing portion and the upper housing portion comprises a third magnet arranged to be magnetically attracted by the first magnet in the closed condition of the upper housing portion. The third magnet may be arranged in a position close to the first magnetic-field sensor. The magnetic attraction of the first magnet, provided on said one of the lower housing portion and the upper housing portion, and the third magnet, provided on said other one of the lower housing portion and the upper housing portion, provides a solid closing force maintaining the upper housing portion in its closed condition relative to the lower housing portion. Said other one of the lower housing portion and the upper housing portion may be provided with a fourth magnet, arranged to be magnetically attracted by the second magnet provided on said one of the lower housing portion and the upper housing portion in the closed condition of the upper housing portion, to provide an additional closing force for maintaining the upper housing portion in its closed condition. The fourth magnet may be arranged in a position close to the second magnetic-field sensor.

In a particular embodiment of the auxiliary device according to the invention, the auxiliary device comprises a rechargeable battery configured and arranged to power the at least one light source and to charge the battery of the personal care device in the predefined stored position of the personal care device. In this way, the charging and disinfecting processes of the auxiliary device may be performed in the absence of an external electrical power source.

The present invention further provides a personal care system comprising an electric personal care device and an auxiliary device according to the invention or any of its embodiments as described herein before, which is configured to store the personal care device, to electrically charge a battery of the personal care device, and to disinfect a personal care unit of the personal care device by means of disinfecting light, wherein:
- the personal care device comprises a main body accommodating the battery and having a longitudinal axis which extends from a first end portion of the main body to a second end portion of the main body;
- the first end portion is provided with an electric charging contact for charging the battery;
- the personal care unit is coupled to the second end portion;
- the main body has a first portion which includes the first end portion, extends along the longitudinal axis from the first end portion towards the second end portion, and excludes a second portion of the main body, said second portion including the second end portion and extending along the longitudinal axis from the second end portion towards the first end portion; and
- the main body and the second portion of the main body have, respectively, a first average length and a second average length measured parallel to the longitudinal axis.

In a particular embodiment of the personal care system according to the invention, the second portion of the main body of the personal care device comprises a light indicator; the personal care device comprises a processor configured to receive an electric communication signal from a processor of the auxiliary device via the electric charging contacts of the auxiliary device and the personal care device when mutually engaging; the processor of the auxiliary device is configured to transmit, via said electric charging contacts when mutually engaging, a predefined communication signal to the processor of the personal care device in reply to at least one of the following: placement of the personal care device in the predefined stored position, and termination of the closed condition of the upper housing portion of the auxiliary device detected by a sensor of the auxiliary device; and the processor of the personal care device is configured to control the light indicator to provide a predefined light output in reply to receipt of said predefined communication signal.

Because, in the predefined stored position of the personal care device within the lower housing portion of the auxiliary device, the second portion of the main body of the personal care device protrudes from the lower housing portion, said light indicator provided on the second portion of the main body remains visible for the user in the opened condition of the upper housing portion. Said light indicator may be annular and arranged circumferentially about the second end portion of the main body. The light indicator may be used to indicate, by means of different colors of light, the pressure exerted on the personal care unit during use of the personal care device. In this particular embodiment, the light indicator may also be used to indicate the correct placement of the personal care device in the predefined stored position within the lower housing portion of the auxiliary device. Said correct placement may be communicated, via the mutually engaging charging contacts, to the processor of the personal care device by means of a dedicated predefined communication signal provided by the processor of the auxiliary device, and the processor of the personal care device may activate the light indicator to provide a dedicated predefined light output in reply to receipt of the communication signal. Alternatively or in addition, the light indicator may be used to indicate, by means of the same or another dedicated light output, the completion of the disinfecting process. Said completion of the disinfecting process may be communicated to the processor of the personal care device by means of a dedicated predefined communication signal provided by the processor of the auxiliary device when termination of the closed condition of the upper housing portion of the auxiliary device is detected by a dedicated sensor of the auxiliary device. Thus, the light indicator on the second portion of the main body may indicate the completion of the disinfecting process when the user opens the auxiliary device by moving the upper housing portion out of its closed condition.

In a further particular embodiment of the personal care system according to the invention, the first portion of the main body of the personal care device comprises a display; the personal care device comprises a processor configured to receive an electric communication signal from a processor of the auxiliary device via the electric charging contacts of the auxiliary device and the personal care device when mutually engaging; the processor of the auxiliary device is configured to transmit, via said electric charging contacts when mutually engaging, a communication signal representing a status of charging the battery of the personal care device and/or disinfecting the personal care unit of the personal care device by means of the auxiliary device; and the processor of the personal care device is configured to control the display to display information relating to said status of charging and/or disinfecting, represented by the transmitted communication signal, upon indication by the transmitted communication signal of removal of the personal care device from the predefined stored position.

Because, in the predefined stored position of the personal care device within the lower housing portion of the auxiliary device, the first portion of the main body of the personal care device is received by the first receiving chamber in the lower housing portion, said display provided on the first portion of the main body is not visible for the user in the predefined stored position of the personal care device. In this particular embodiment the processor of the personal care device may receive from the processor of the auxiliary device, via the communication signal exchanged via the mutually engaging electric charging contacts, information about the status of the charging process of the battery of the personal care device and/or the disinfecting process of the personal care unit of the personal care device. When said communication signal indicates the removal of the personal care device from the predefined stored position, i.e. interruption of the mutual engagement of the electric charging contacts, the processor of the personal care device may control the display to display information relating to said status of the charging process and/or the disinfecting process, represented by the transmitted communication signal. Thus, when the user removes the personal care device from the auxiliary device and the display on the first portion of the main body of the personal care device becomes visible again for the user, the display may show the result of the charging process, e.g., the charging level of the battery of the personal care device, and the result of the disinfecting process, e.g., completion of the disinfecting process.

In a further embodiment of the personal care system according to the invention, wherein the auxiliary device comprises a rechargeable battery, the transmitted communication signal further represents a status of the rechargeable battery of the auxiliary device, and the processor of the personal care device is configured to control the display to further display information relating to said status of the rechargeable battery of the auxiliary device, represented by the transmitted communication signal, upon indication by the transmitted communication signal of removal of the personal care device from the predefined stored position. In this further embodiment, when the user removes the personal care device from the auxiliary device and the display on the first portion of the main body of the personal care device becomes visible again for the user, the display may also show the status, e.g., the charging level, of the rechargeable battery of the auxiliary device.

In a yet further embodiment of the personal care system according to the invention, the processor of the auxiliary device is configured to transmit, via said electric charging contacts when mutually engaging, a predefined communication signal to the processor of the personal care device in reply to placement of the personal care device in the predefined stored position; and the processor of the personal care device is configured to deactivate the display in reply to receipt of said predefined communication signal. In this yet further embodiment, the display provided on the first portion of the main body of the personal care device is automatically deactivated when the user places the personal care device in its predefined stored position within the lower housing portion of the auxiliary device and the display is no longer visible for the user. This will save energy consumption of the personal care system and may extend the lifetime of the display.

The above-described and other aspects of the invention will be apparent from and elucidated with reference to the following detailed description of embodiments of an auxiliary device and a personal care system in accordance with the invention.

### BRIEF DESCRIPTION OF THE DRAWINGS

The invention will be explained in greater detail with reference to the figures, in which equal or similar features are indicated by the same reference numbers, and in which:
Fig. 1 shows an embodiment of a personal care system according to the invention;
Fig. 2 shows a side view of the personal care system of Fig. 1;
Fig. 3 shows a personal care device, in particular an electric shaver, of the personal care system of Fig. 1;
Fig. 4 shows an embodiment of an auxiliary device according to the invention which is part of the personal care system of Fig. 1;
Fig. 5 shows a rear view of the auxiliary device of Fig. 4;
Fig. 6 shows a longitudinal cross-section of the personal care system of Fig. 1 in an opened condition of an upper housing portion of the auxiliary device;
Fig. 7 shows a top view of the auxiliary device of Fig. 4 in the opened condition of the upper housing portion of the auxiliary device;
Fig. 8 shows a longitudinal cross-section of the personal care system of Fig. 1 in a closed condition of the upper housing portion of the auxiliary device;
Fig. 9 is a detailed view on the upper housing portion of the personal care system of Fig. 1;
Fig. 10 shows in detail an upper part of the longitudinal cross-section of Fig. 8;
Fig. 11a shows an electric charging contact of the auxiliary device of Fig. 4;
Fig. 11b shows a cross-section of the electric charging contact of Fig.11a; and
Fig. 12 shows a control circuitry of the auxiliary device of Fig. 4; and
Fig. 13 schematically shows a control system of the personal care system of Fig. 1 for controlling a light indicator and a display of the personal care device of Fig. 3.

### DETAILED DESCRIPTION OF THE EMBODIMENTS

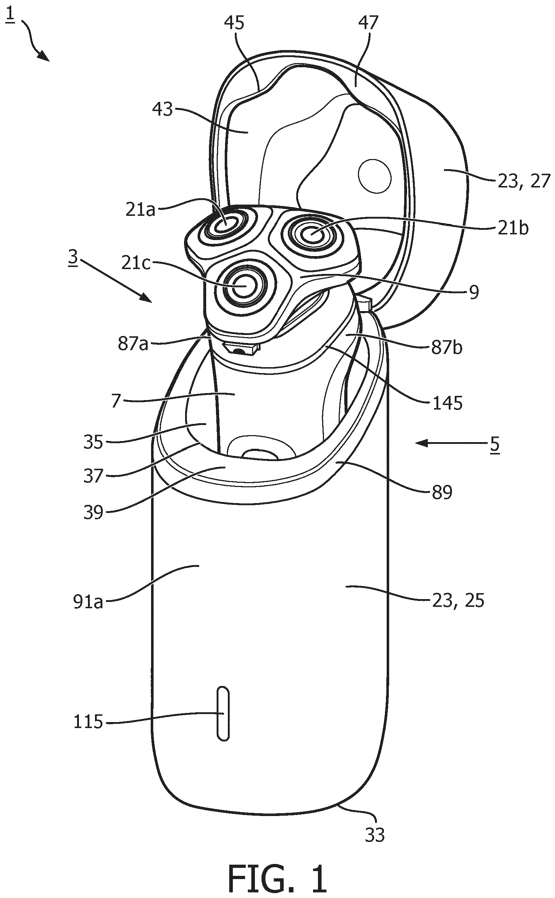

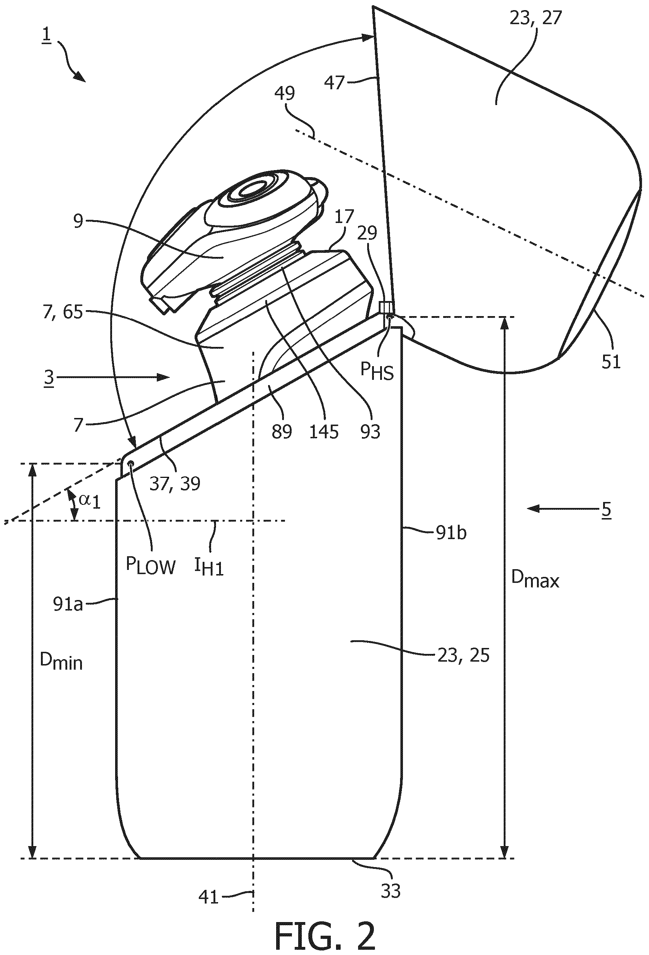

Figs. 1 and 2 show an embodiment of a personal care system 1 according to the invention. The personal care system 1 comprises an electric personal care device 3 and an embodiment of an auxiliary device 5 according to the invention. The personal care device 3 comprises a main body 7 and a personal care unit 9 which is coupled to the main body 7. In the embodiment of Figs. 1 and 2, the personal care device 3 is an electric shaver and the personal care unit 9 is a shaving unit. The auxiliary device 5 is configured to store the personal care device 3, to electrically charge a battery of the personal care device 3, and to disinfect the personal care unit 9 of the personal care device 3 by means of disinfecting light.

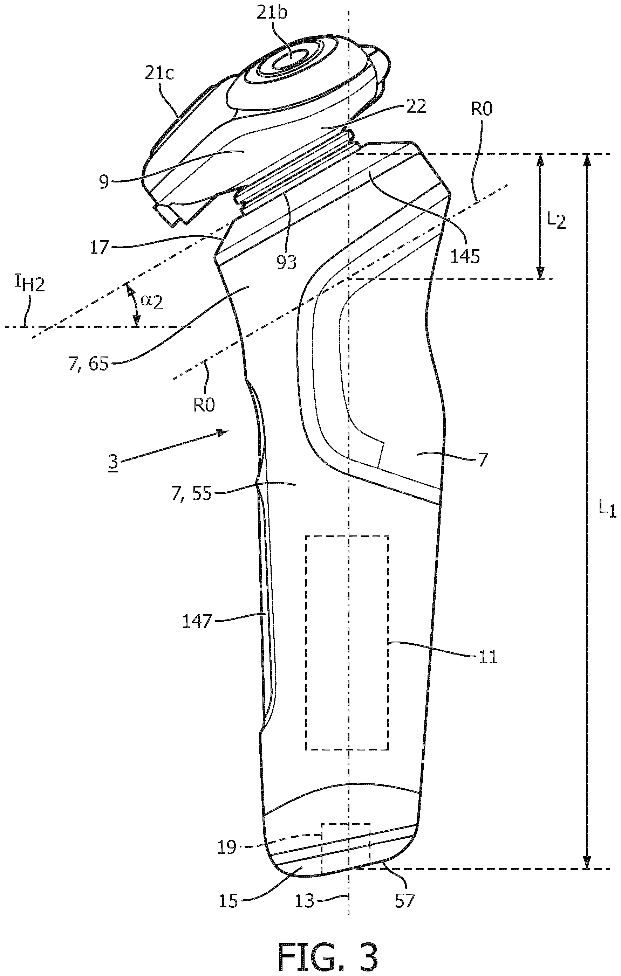

In Fig. 3, the personal care device 3, i.e., the electric shaver, of the personal care system 1 is shown separately from the auxiliary device 5. The main body 7 of the personal care device 3 accommodates the battery 11 of the personal care device 3. The battery 11 is a rechargeable battery, and its position in the main body 7 is only schematically shown. The main body 7 has a longitudinal axis 13 which extends from a first end portion 15 of the main body 7 to a second end portion 17 of the main body 7. The first end portion 15 of the main body 7 is provided with an electric charging contact 19 for charging the battery 11. The charging contact 19 of the personal care device 3 is only schematically shown in Fig. 3 and may be of any suitable type well known to the skilled person. The personal care unit 9, i.e., the shaving unit, is coupled to the second end portion 17 of the main body 7. In the embodiment shown in the figures, the shaving unit comprises three circularly shaped hair-cutting units 21a, 21b, 21c which are arranged triangularly on a supporting member 22 of the shaving unit. The hair-cutting units 21a, 21b, 21c are of a rotary type and each comprise an external cutting member with an annular array of hair-entry openings and an internal cutting member with an annular array of cutting elements, which is covered by and rotatable relative to the external cutting member as is well known to the skilled person. The shaving unit may be fixedly or releasably coupled to the main body 7, as is also well known to the skilled person. When the shaving unit is coupled to the main body 7, the internal cutting members of the three hair-cutting units 21a, 21b, 21c are rotatable by means of a motor of the personal care device 3, not shown in Fig. 3, arranged in the main body 7.

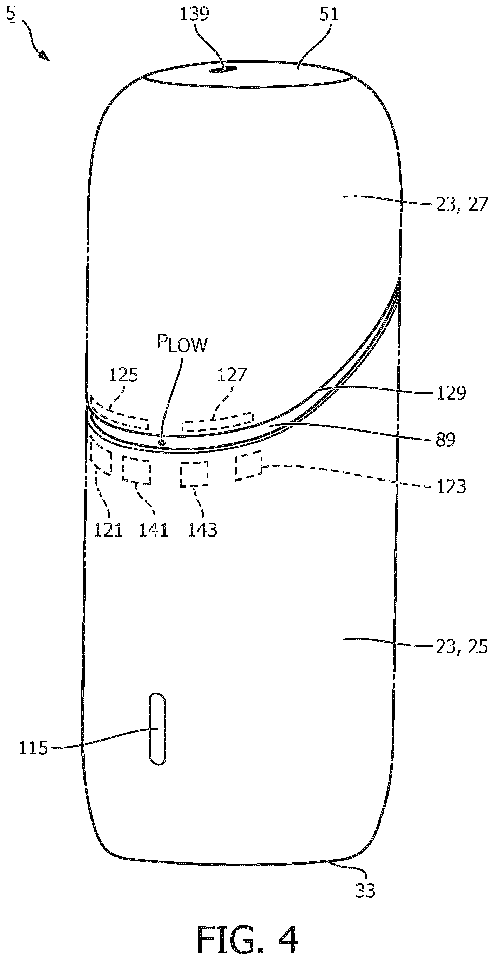

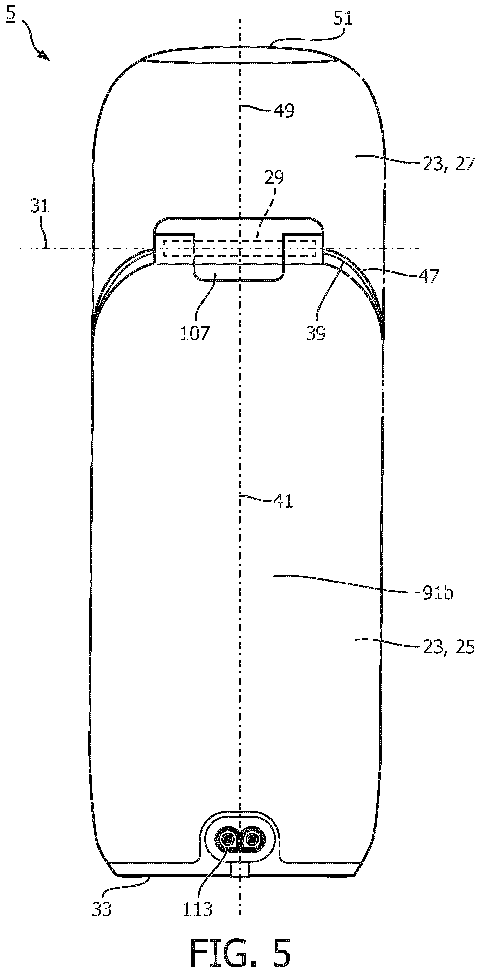

In Fig. 4, the auxiliary device 5 of the personal care system 1 is shown separately. As shown in Figs. 1, 2 and 4, the auxiliary device 5 comprises a housing 23 having a lower housing portion 25 and an upper housing portion 27. The upper housing portion 27 has a closed condition and an opened condition relative to the lower housing portion 25. The closed condition of the upper housing portion 27 is shown in Fig. 4, and the opened condition of the upper housing portion 27 is shown in Figs. 1 and 2. In the embodiment of the auxiliary device 5 shown in the figures, the upper housing portion 27 is pivotable relative to the lower housing portion 25 from the closed condition to the opened condition, and vice versa, by means of a hinge structure 29. In particular, the upper housing portion 27 is manually pivotable by a user of the personal care system 1. The hinge structure 29 is only schematically shown in Fig. 5, which represents a rear view of the auxiliary device 5. The hinge structure 29 may be of any suitable type well known to the skilled person and defines a pivot axis 31 about which the upper housing portion 27 is pivotable relative to the lower housing portion 25.

Figs. 1, 2, 4 and 5 show the auxiliary device 5 in a normal operational position and orientation. As particularly shown in Figs. 2 and 5, in said normal operational position and orientation of the auxiliary device 5 a bottom side 33 of the lower housing portion 25 is supportable in a horizontal orientation by an external horizontal supporting surface, e.g., a horizontally oriented table surface. As a result, the normal operational position and orientation of the auxiliary device 5 may be defined by the horizontal orientation of the bottom side 33 of the lower housing portion 25. The bottom side 33 may comprise a flat surface supportable by an external horizontal supporting surface. Alternatively, the bottom side 33 may comprise a plurality of supporting feet configured and arranged to define the horizontal orientation of the bottom side 33 when jointly supported by an external horizontal supporting surface. As shown in Fig. 1, the lower housing portion 25 of the auxiliary device 5 further comprises a first receiving chamber 35. In the opened condition of the upper housing portion 27, the first receiving chamber 35 is accessible via a receiving opening 37 which is provided at an upper side 39 of the lower housing portion 25. As shown in Figs. 2 and 5, the lower housing portion 25 further has a longitudinal axis 41 which, in the normal operational position and orientation of the auxiliary device 5, extends in a vertical direction from the bottom side 33 to the upper side 39 of the lower housing portion 25. Thus, alternatively, the normal operational position and orientation of the auxiliary device 5 may be defined by the vertical orientation of the longitudinal axis 41 of the lower housing portion 25.

As shown in Fig. 1, the upper housing portion 27 of the auxiliary device 5 comprises a second receiving chamber 43. In the opened condition of the upper housing portion 27, the second receiving chamber 43 is accessible via a receiving opening 45 which is provided at a lower side 47 of the upper housing portion 27. As shown in Fig. 2, the upper housing portion 27 has a longitudinal axis 49 which extends from the lower side 47 to a top side 51 of the upper housing portion 27. In the closed condition of the upper housing portion 27, as shown in Fig. 5, the longitudinal axis 41 of the lower housing portion 25 and the longitudinal axis 49 of the upper housing portion 27 are in line with each other. Thus, in the normal operational position and orientation of the auxiliary device 5 with the upper housing portion 27 being in its closed condition as shown in Figs. 4 and 5, the lower housing portion 25 and the upper housing portion 27 form a vertically standing housing 23 of the auxiliary device 5 having a slim elongated shape extending in the vertical direction.

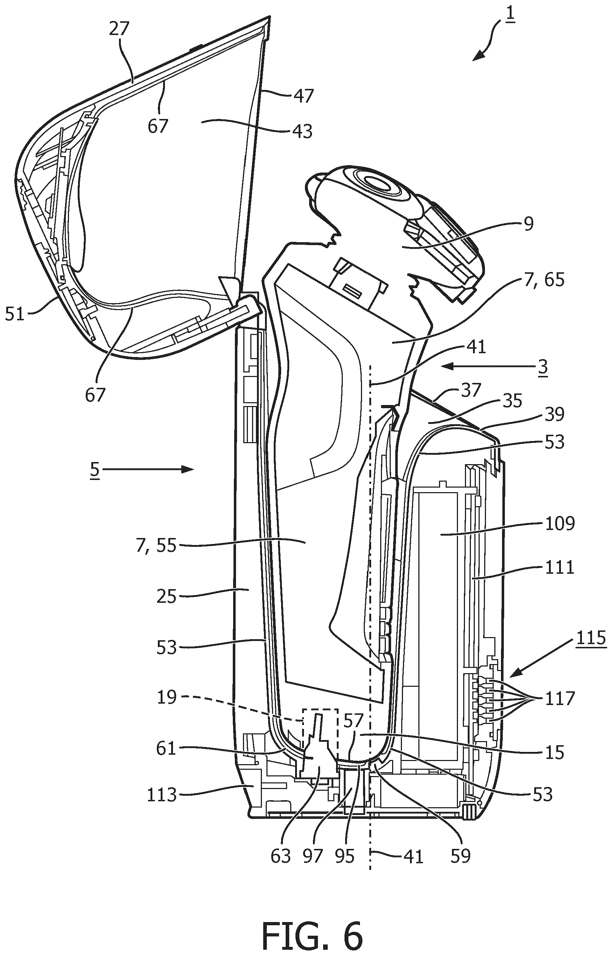

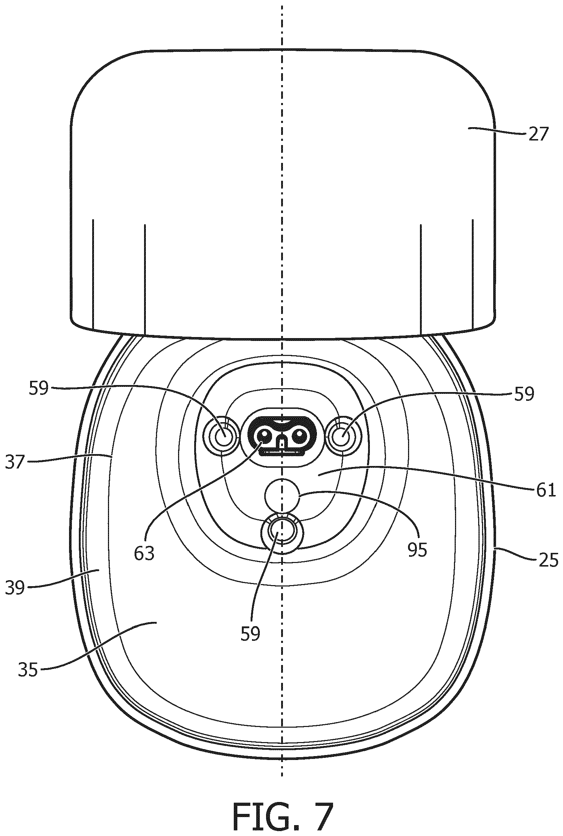

Fig. 6 shows a longitudinal cross-section of the personal care system 1 in the opened condition of the upper housing portion 27 of the auxiliary device 5. As shown in Fig. 6, the first receiving chamber 35 in the lower housing portion 25 is enclosed by an inner shell structure 53 of the lower housing portion 25. The first receiving chamber 35 is configured to receive a first portion 55 of the main body 7 of the personal care device 3 in a predefined stored position of the personal care device 3. In said predefined stored position a lower side 57 of the main body 7, which is provided at the first end portion 15 of the main body 7, is supported by three supporting elements 59 which are provided on a bottom surface 61 of the first receiving chamber 35. The three supporting elements 59 are visible in Fig. 7, which is a top view of the auxiliary device 5 in the opened condition of the upper housing portion 27. Fig. 6 shows one of the three supporting elements 59 in supporting contact with the lower side 57 of the main body 7.

As further shown in Figs. 6 and 7, the lower housing portion 25 of the auxiliary device 5 comprises an electric charging contact 63 which is arranged to engage the electric charging contact 19 of the main body 7 of the personal care device 3 in the predefined stored position of the personal care device 3. In the embodiment shown in the figures, the electric charging contact 19 of the main body 7 is provided on the lower side 57 of the main body 7, and the electric charging contact 63 of the auxiliary device 5 is provided on the bottom surface 61 of the first receiving chamber 35 in the lower housing portion 25. As further shown in Fig. 6, the first receiving chamber 35, i.e., the inner shell structure 53 of the lower housing portion 25, tapers in a direction along the longitudinal axis 41 of the lower housing portion 25 from the receiving opening 37 towards the bottom surface 61. When the user places the personal care device 3 via the receiving opening 37 into the first receiving chamber 35 of the lower housing portion 25, said tapering of the first receiving chamber 35 enables the user to correctly place the personal care device 3 into the predefined stored position, with the lower side 57 of the main body 7 being supported by the three supporting elements 59 provided on the bottom surface 61 of the first receiving chamber 35, and further enables the electric charging contact 19 of the personal care device 3 to be correctly aligned for engagement with the electric charging contact 63 of the auxiliary device 5. Furthermore, the arrangement of the electric charging contact 19 on the lower side 57 of the main body 7 of the personal care device 3 and the arrangement of the electric charging contact 63 on the bottom surface 61 of the first receiving chamber 35 of the auxiliary device 5 enable a solid and secure engagement of the electric charging contacts 19 and 63 under the influence of gravity force during and after placement of the personal care device 3 into the lower housing portion 25.

As further shown in Figs. 2 and 6, the lower housing portion 25 of the auxiliary device 5 is configured such that, in the opened condition of the upper housing portion 27 and in the predefined stored position of the personal care device 3, a second portion 65 of the main body 7 of the personal care device 3 protrudes from the lower housing portion 25 via the receiving opening 37 in the upper side 39 of the lower housing portion 25. The first portion 55 and the second portion 65 of the main body 7 of the personal device 3 are also indicated in Fig. 3, wherein the line RO indicates the position of the receiving opening 37 of the lower housing portion 25 relative to the main body 7 when the personal care device 3 is in the predefined stored position within the first receiving chamber 35 in the lower housing portion 25. As shown in Fig. 3, the first portion 55 of the main body 7 includes the first end portion 15 of the main body 7, which is provided with the electric charging contact 19. The first portion 55 extends along the longitudinal axis 13 of the main body 7 from the first end portion 15 towards the second end portion 17 of the main body 7, but excludes the second portion 65 of the main body 7. As shown in Fig. 3, the second portion 65 of the main body 7 includes the second end portion 17 of the main body 7, to which the personal care unit 9 is coupled. The second portion 65 extends along the longitudinal axis 13 of the main body 7 from the second end portion 17 towards the first end portion 15. As further shown in Fig. 3, the main body 7 has a first average length L₁ measured parallel to the longitudinal axis 13 of the main body 7, and the second portion 65 of the main body 7 has a second average length L₂ measured parallel to the longitudinal axis 13 of the main body 7.

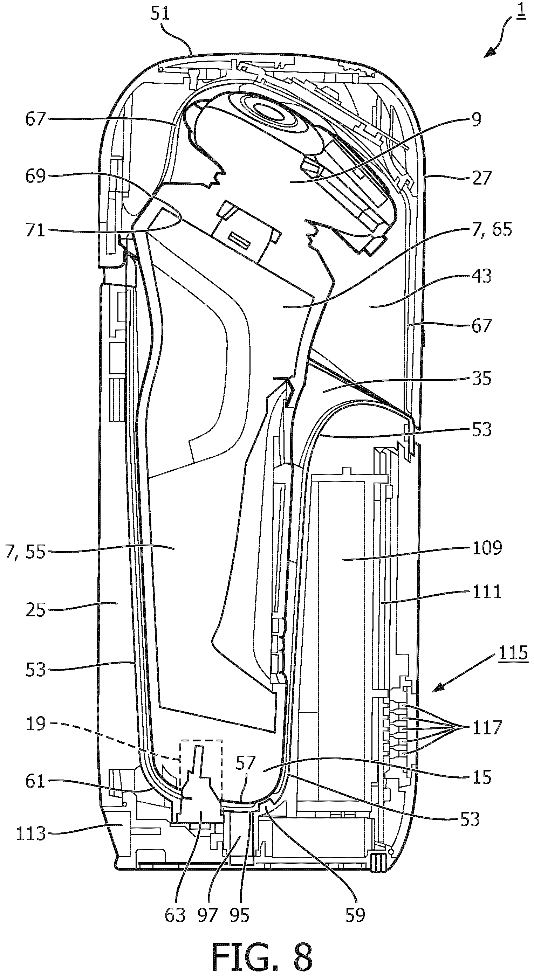

Starting from the opened condition of the upper housing portion 27, with the personal care device 3 being placed in the predefined stored position within the first receiving chamber 35 of the lower housing portion 25 as shown in Figs. 1, 2 and 6, the user may pivot the upper housing portion 27 into its closed condition as shown in Figs. 4, 5 and 8. As shown in Fig. 8, which is a longitudinal cross-section of the personal care system 1 in the closed condition of the upper housing portion 27 of the auxiliary device 5, the second receiving chamber 43 in the upper housing portion 27 is configured to receive, in the closed condition of the upper housing portion 27 with the personal care device 3 being placed in the predefined stored position, the second portion 65 of the main body 7 of the personal care device 3 and the personal care unit 9 coupled to the main body 7. As shown in Fig. 8, the second receiving chamber 43 is enclosed by an inner shell structure 67 of the upper housing portion 27. The inner shell structure 67 comprises a supporting surface 69 which is arranged to locally contact a rear surface portion 71 of the second portion 65 of the main body 7 of the personal care device 3 in the closed condition of the upper housing portion 27. The supporting surface 69 in the second receiving chamber 43 and the three supporting elements 59 on the bottom surface 61 of the first receiving chamber 35 together provide a stable support of the personal care device 3 in its predefined stored position and in the closed condition of the upper housing portion 27.

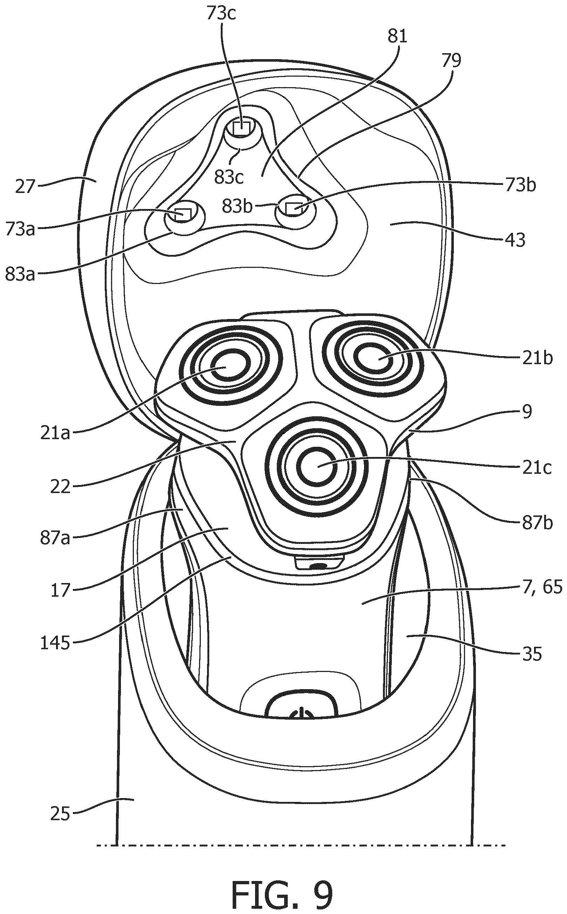

The upper housing portion 27 comprises at least one light source configured to generate disinfecting light and arranged to expose the personal care unit 9 with the disinfecting light in the closed condition of the upper housing portion 27 and in the predefined stored position of the personal care device 3. The disinfecting light may comprise ultraviolet, UV, disinfecting light and/or another wavelength of light having disinfecting properties, such as blue light. In the embodiment of the personal care system 1 shown in the figures, wherein the personal care device 3 is an electric shaver and wherein the personal care unit 9 is a shaving unit comprising the three circularly shaped hair-cutting units 21a, 21b, 21c arranged triangularly on the supporting member 22 of the shaving unit as described herein before, the upper housing portion 27 comprises three light sources 73a, 73b, 73c, in particular three UV-LEDs. As shown in Fig. 9, which provides a detailed view on the upper housing portion 27 in its opened condition, the three light sources 73a, 73b, 73c are arranged triangularly to each mainly expose a respective one of the three hair-cutting units 21a, 21b, 21c of the shaving unit with the disinfecting light in the closed condition of the upper housing portion 27 and in the predefined stored position of the electric shaver. For this purpose, in the closed condition of the upper housing portion 27, each of the three light sources 73a, 73b, 73c is arranged adjacent to a respective one of the three hair-cutting units 21a, 21b, 21c of the shaving unit.

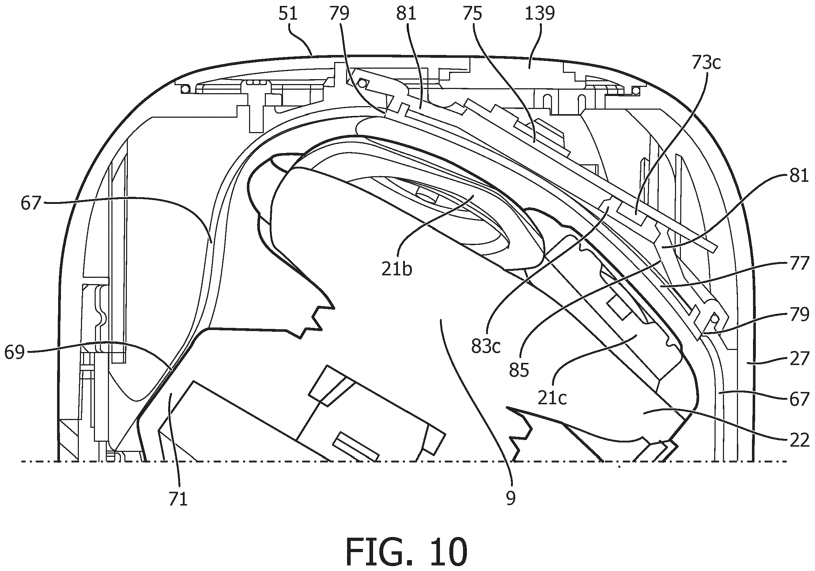

In the cross-section of Fig. 10, the position of the light source 73c adjacent to the hair-cutting unit 21c in the closed condition of the upper housing portion 27 is visible in detail. The positions of the light sources 73a, 73b adjacent to, respectively, the hair-cutting units 21a, 21b is similar. As further shown in Fig. 10, the light sources 73a, 73b, 73c are mounted on a printed circuit board, PCB, 75 which is arranged between the inner shell structure 67 and the top side 51 of the upper housing portion 27. The disinfecting light generated by the light sources 73a, 73b, 73c is transmitted to the hair-cutting units 21a, 21b, 21c via an optically transparent window 77, which is arranged in a corresponding opening 79 within the inner shell structure 67 of the upper housing portion 27. Furthermore, a reflector 81 is arranged between the PCB 75 and the window 77. The reflector 81 has a respective opening 83a, 83b, 83c for each of the respective light sources 73a, 73b, 73c wherein the respective light source 73a, 73b, 73 is arranged. In Fig. 10, only the opening 83c associated with the light source 73c is visible. In Fig. 9, the light sources 73a, 73b, 73c, the reflector 81 and the openings 83a, 83b, 83c in the reflector 81 are visible through the transparent window 77, which is not visible in Fig. 9. A side of the reflector 81 facing the window 77 is provided with a coating 85 of an optically reflecting material, e.g., chrome, as indicated in Fig. 10. The triangular arrangement of the three light sources 73a, 73b, 73c in the upper housing portion 27 and the reflector 81 provided with the coating 85 enable an effective and efficient exposure of the three hair-cutting units 21a, 21b, 21c of the shaving unit to the disinfecting light.

As shown in Fig. 8, the lower housing portion 25 and the upper housing portion 27 of the auxiliary device 5, i.e., in particular the first receiving chamber 35 and the inner shell structure 53 of the lower housing portion 25 and the second receiving chamber 43 and the inner shell structure 67 of the upper housing portion 27, are configured to fully enclose the main body 7 and the personal care unit 9 of the personal care device 3 in the closed condition of the upper housing portion 27 and in the predefined stored position of the personal care device 3. In the closed condition of the upper housing portion 27, the first receiving chamber 35 and the second receiving chamber 43 jointly form a fully closed cavity. This will prevent, in the closed condition of the upper housing portion 27, leakage of the disinfecting light generated by the light sources 73a, 73b, 73c to the environment of the auxiliary device 5 and, thus, will prevent any safety risks for the user, in particular for the user's eyes. In addition, said fully closed cavity, formed by the first and second receiving chambers 35, 43 in the closed condition of the upper housing portion 27, will protect the personal care device 3 against any external loads or other types of external impacts, e.g. during transportation of the auxiliary device 5 and the personal care device 3 stored therein.

As explained herein before and as shown in the figures, the lower housing portion 25 and the upper housing portion 27 of the auxiliary device 5 are configured to receive the personal care device 3 in a substantial vertical, i.e., upright standing orientation. For this purpose, as described herein before, the bottom side 33 of the lower housing portion 25 is configured to be supportable in a horizontal orientation, such that the longitudinal axis 41 of the lower housing portion 25 and the longitudinal axis 49 of the upper housing portion 27 (in the closed condition) extend in a vertical direction in the normal operational position and orientation of the auxiliary device 5. This allows the lower housing portion 25 and the upper housing portion 27 to have dimensions in directions perpendicular to their longitudinal axes 41, 49 which are small relative to a total height of the housing 23 of the auxiliary device 5 measured parallel to said longitudinal axes 41, 49. In particular, the dimensions of the lower and upper housing portions 25, 27 perpendicular to their longitudinal axes 41, 49 may be minimized by a slim design of the inner shell structures 53, 67 of the lower and upper housing portions 25, 27, as shown in Figs. 6 and 8, wherein the inner shell structures 53, 67 closely surround, respectively, the first portion 55 of the main body 7 and the second portion 65 of the main body 7 with the personal care unit 9 coupled thereto. Thereby, in particular the bottom side 33 of the lower housing portion 25, by which the auxiliary device 5 is to be supported by a horizontal external supporting surface, e.g., a horizontal table surface, may have relatively small dimensions, so that the auxiliary device 5 may occupy only a relatively small surface area when placed on said horizontal supporting surface.

Furthermore, the upright standing orientation of the lower housing portion 25 in the normal operational position of the auxiliary device 5 allows the user to conveniently place and remove, in the opened condition of the upper housing portion 27, the main body 7 of the personal care device 3 via the receiving opening 37 at the upper side 39 of the lower housing portion 25 into and from its predefined stored position within the first receiving chamber 35 of the lower housing portion 25. Because, in the predefined stored position of the auxiliary device 5, the second portion 65 of the main body 7 protrudes from the lower housing portion 25 via the receiving opening 37 as shown in Figs. 1 and 2, the user is enabled to conveniently grasp the main body 7 at its second portion 65, without touching the personal care unit 9, during placing and removing of the personal care device 3 into and from the lower housing portion 25 of the auxiliary device 5. For this purpose, in accordance with the invention, a ratio (L₂/L₁) between the second average length L₂ of the second portion 65 and the first average length L₁ of the main body 7, both as defined herein before and as shown in Fig. 3, is at least 0.05. For most commonly used personal care devices having a main body with an elongated shape, this ratio allows the user sufficient space to easily grasp the second portion 65 of the main body 7 by means of at least two fingers placed at two mutually opposite positions 87a, 87b at the circumferential surface of the second portion 65 of the main body 7 when protruding from the lower housing portion 25, as indicated in Figs. 1 and 9. More preferably, the ratio L₂/L₁ is from 0.1 to 0.35. A ratio L₂/L₁ within this preferred range allows the user to more conveniently and intuitively grasp the second portion 65 of the main body 7 when protruding from the lower housing portion 25, and also still allows, in the opened condition of the upper housing portion 27, a stable support of the first portion 55 of the main body 7 within the first receiving chamber 35 in the lower housing portion 25 after placement of the personal care device 3 into the predefined stored position. Said stable support is required in order to allow the upper housing portion 27 to pivot from the opened condition into the closed condition without being obstructed by an incorrect position of the personal care device 3 in the lower housing portion 25. In the embodiment of the personal care system 1 shown in the figures, the ratio L₂/L₁ is about 0.17.

As further shown in Figs. 1 and 2, in the embodiment of the auxiliary device 5 shown in the figures a main orientation of the receiving opening 37 of the lower housing portion 25 is oblique relative to the longitudinal axis 41 of the lower housing portion 25. In particular, Fig. 2 shows a sharp inclination angle α₁ which is enclosed by the receiving opening 37 and an imaginary horizontal plane I_{H1} in the normal operational position and orientation of the auxiliary device 5. Figs. 1 and 2 further show an edge structure 89 of the lower housing portion 25, which is provided circumferentially around the receiving opening 37. Fig. 2 further shows that the hinge structure 29, by means of which the upper housing portion 27 is pivotable from its closed condition to its opened condition, and vice versa, is provided in a position P_{HS} on the edge structure 89 of the lower housing portion 25. In said position P_{HS} the edge structure 89 has a largest distance D_{MAX} from the bottom side 33 of the lower housing portion 25, measured in a direction parallel to the longitudinal axis 41 of the lower housing portion 25. As shown in Fig. 2, as a result of the inclined orientation of the receiving opening 37 of the lower housing portion 25, the edge structure 89 has a smallest distance D_{MIN} from the bottom side 33 of the lower housing portion 25 at a lowest position P_{LOW} on the edge structure 89 which is diametrically opposite to the position P_{HS} relative to the longitudinal axis 41 of the lower housing portion 25. A shown in Figs. 1 and 2, the inclined orientation of the receiving opening 37 and the arrangement of the hinge structure 29 in said position P_{HS} result in an increased visibility and accessibility of the personal care device 3 from a front side 91a of the auxiliary device 5, which is opposite to a rear side 91b where the hinge structure 29 is present. Furthermore, as shown in Fig. 2, the arrangement of the hinge structure 29 in said position P_{HS} minimizes the risk for the upper housing portion 27 of being obstructed by the personal care device 3 when pivoting relative to the lower housing portion 25.

In the embodiment of the personal care system 1 as shown in the figures, an upper side 93 of the main body 7 of the personal care device 3, which is provided at the second end portion 17 of the main body 7, has a main orientation which is oblique relative to the longitudinal axis 13 of the main body 7. In particular, Fig. 3 shows a sharp inclination angle α₂ which is enclosed by the upper side 93 of the main body 7 and an imaginary plane I_{H2} extending perpendicularly to the longitudinal axis 13 of the main body 7. As shown in Fig. 2, in the predefined stored position of the personal care device 3 within the lower housing portion 25 of the auxiliary device 5, the main orientation of the receiving opening 37 of the lower housing portion 25 is substantially parallel to the main orientation of the upper side 93 of the main body 7. As a result, a distance over which the second portion 65 of the main body 7 protrudes from the lower housing portion 25 in the opened condition of the upper housing portion 27 is substantially identical along a circumference of the second portion 65. This will further improve the convenience for the user of grasping the second portion 65 of main body 7 during placing and removing the personal care device 3 into and from the lower housing portion 25.

As shown in Figs. 6, 7 and 8, in the embodiment of the personal care system 1 a drainage opening 95 is provided at the bottom surface 61 of the first receiving chamber 35 in the lower housing portion 25, i.e., in a lowermost position within the auxiliary device 5. Via said drainage opening 95, water present in the first receiving chamber 35 may flow out of the auxiliary device 5 via a drainage channel 97 that connects the drainage opening 95 with an opening at the bottom side 33 of the lower housing portion 25. Water or cleaning liquid may drip from the personal care device 3 when the user has cleaned the personal care device 3, in particular the personal care unit 9, before placing the personal care device 3 into the auxiliary device 5, and may thus collect at the bottom surface 61 of the first receiving chamber 35 and flow out of the auxiliary device 5 via the drainage channel 97.

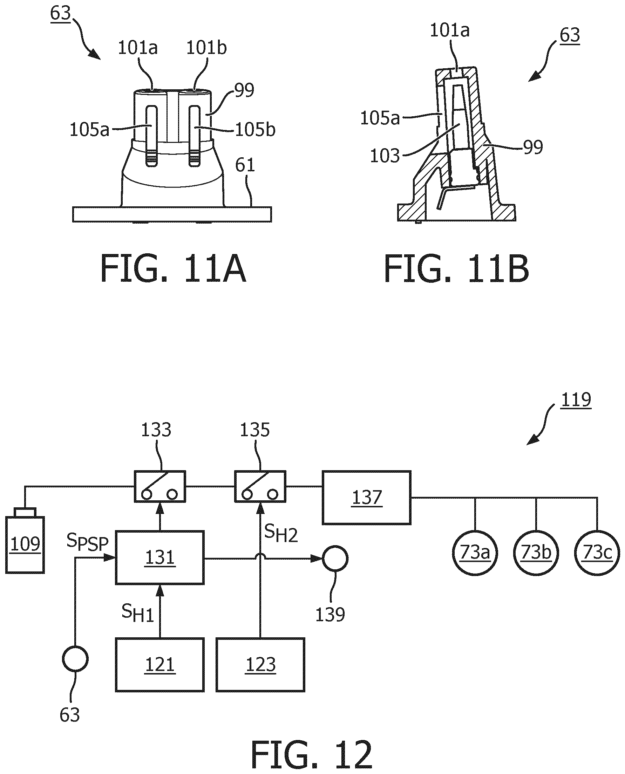

Water or cleaning liquid dripping from the personal care device 3 may also moisten the electric charging contact 63 of the auxiliary device 5, which is provided on the bottom surface 61 of the first receiving chamber 35. The electric charging contact 63 is shown in detail in Fig. 11a, and Fig. 11b shows a cross-section of the electric charging contact 63. The electric charging contact 63 comprises a plastic plug housing 99 provided with two plug openings 101a, 101b on its top side, via which two metal electric contact elements 103 are accessible. In Fig. 11b one of the metal electric contact elements 103, which is accessible via the plug opening 101a, is visible. Water can penetrate into the plug housing 99 via the plug openings 101a, 101b and may moisten the electric contact elements 103. As shown in Fig. 11a, the plug housing 99 is provided with two drainage slots 105a, 105b, via which any water may flow out of the plug housing 99 and may flow further into the drainage opening 95 in the bottom surface 61 of the first receiving chamber 35. In Fig. 11b, the drainage slot 105a is visible in cross-section.

Furthermore, to accelerate drying of the personal care device 3 within the auxiliary device 5 in the closed condition of the upper housing portion 27, a ventilation opening 107 is provided at the rear side 91b of the lower housing portion 25 below the hinge structure 29, as shown in Fig. 5.

As is further shown in Figs. 6 and 8, in the embodiment of the personal care system 1 the auxiliary device 5 comprises a rechargeable battery 109. The rechargeable battery 109 is provided on a main printed circuit board, main PCB, 111 which is arranged in the lower housing portion 25. The main PCB 111 comprises electrical power circuitry, not shown in the figures, configured to power the light sources 73a, 73b, 73c by means of power from the rechargeable battery 109. For this purpose, the light sources 73a, 73b, 73c and the PCB 75 in the upper housing portion 27 are electrically connected to the main PCB 111 in the lower housing portion 25 by means of electric wires, not shown in the figures, which pass through the hinge structure 29. The electrical power circuitry of the main PCB 111 is further configured to charge the battery 11 of the personal care device 3 by means of power from the rechargeable battery 109 in the predefined stored position of the personal care device 3, i.e., when the electrical charging contacts 19, 63 of the personal care device 3 and the auxiliary device 5 mutually engage. Thus, the auxiliary device 5 is able to disinfect the personal are unit 9 of the personal care device 3 and to charge the battery 11 of the personal care device 3 also in the absence of an external electrical power source, e.g., when the user is traveling.

As shown in Figs. 5, 6 and 8, an external charging port 113 is provided on the rear side 91b of the lower housing portion 25 near the bottom side 33. The electrical power circuitry of the main PCB 111 is further configured to charge the rechargeable battery 109 of the auxiliary device 5 by means of electric power provided by an external electrical power source connected to the external charging port 113. In particular, said charging of the rechargeable battery 109 of the auxiliary device 5 is enabled when the personal care device 3 is not stored in the auxiliary device 5 or when the personal care device 3 is stored in the auxiliary device 5 but does not need to be disinfected and/or charged. When the rechargeable battery 109 of the auxiliary device 5 is empty and the personal care device 3 is stored in the auxiliary device 5, the electrical power circuitry of the main PCB 111 may power the light sources 73a, 73b, 73c and/or charge the battery 11 of the personal care device 3 by means of electric power provided by an external electrical power source connected to the external charging port 113. In the latter case, the electrical power circuitry of the main PCB 111 may simultaneously charge the rechargeable battery 109 of the auxiliary device 5, although at a reduced charging power compared to the case wherein the personal care device 3 is not stored in the auxiliary device 5 or the personal care device 3 is stored in the auxiliary device 5 but does not need to be disinfected and/or charged. As shown in Figs. 1, 4, 6 and 8, a first light indicator 115 is arranged on the lower housing portion 25 on the front side 91a of the auxiliary device 5. The first light indicator 115 has a plurality of LEDs 117 arranged on the main PCB 111 and controlled by the electrical power circuitry of the main PCB 111 to step-wise indicate the charging level of the rechargeable battery 109 of the auxiliary device 5, as is well known to the skilled person.

Fig. 12 shows a control circuitry 119 of the auxiliary device 5 of the personal care system 1, which is configured to control the electric power supply to the light sources 73a, 73b, 73c of the auxiliary device 5. Fig. 12 shows the case, as described herein before, wherein the electric power is supplied from the rechargeable battery 109 of the auxiliary device 5. However, the functioning of the control circuitry 119 may be similar in the alternative case wherein the electric power is supplied from an external electrical power source connected to the external charging port 113 of the auxiliary device 5. The control circuitry 119 may be part of the main PCB 111.

The control circuitry 119 comprises a first magnetic-field sensor 121 and a second magnetic-field sensor 123. In the embodiment shown, the first and second magnetic-field sensors 121, 123 each comprise a Hall sensor. As schematically shown in Fig. 4, the first and second magnetic-field sensors 121, 123 are arranged in the lower housing portion 25 of the auxiliary device 5 in two respective positions close to and mutually opposite relative to the lowest position P_{LOW} on the edge structure 89 of the lower housing portion 25. As further shown in Fig. 4, a first magnet 125 and a second magnet 127 are arranged in the upper housing portion 27 in positions close to a lower edge 129 of the upper housing portion 27. The first magnet 125 is arranged to enable the first magnetic-field sensor 121 to detect different magnetic-field strengths in, respectively, the closed condition and the opened condition of the upper housing portion 27. Similarly, the second magnet 127 is arranged to enable the second magnetic-field sensor 123 to detect different magnetic-field strengths in, respectively, the closed condition and the opened condition of the upper housing portion 27. Thus, the first and second magnetic-field sensors 121, 123 are each enabled to provide, based on the magnetic-field strength respectively detected by the first and second magnetic-field sensors 121, 123, a respective output signal S_{H1}, S_{H2}, as shown in Fig. 12, indicating whether or not the upper housing portion 27 is in its closed condition.

The control circuitry 119 further comprises a processor 131. The processor 131 is configured to receive the output signal S_{H1} provided by the first magnetic-field sensor 121. The processor 131 is further configured to receive an electric detection signal S_{PSP}. The detection signal S_{PSP} is received from the electric charging contact 63 of the auxiliary device 5. The detection signal S_{PSP} may be exchanged between the processor 131 and a processor of the personal care device 3 via the electric charging contacts 19, 63 of the personal care device 3 and the auxiliary device 5 when the electric charging contacts 19, 63 mutually engage. Thus, the detection signal S_{PSP} may indicate the presence of the personal care device 3 in the predefined stored position within the lower housing portion 25. The processor 131 is configured to control a first electric switch 133 of the control circuitry 119 in dependence on the output signal S_{H1} and the detection signal S_{PSP}. The first electric switch 133 comprises, e.g., a MOSFET. In particular, the processor 131 is configured to close the first electric switch 133 for a predetermined time period upon detection of both the closed condition of the upper housing portion 27 based on the output signal S_{H1} of the first magnetic-field sensor 121 and the predefined stored position of the personal care device 3 based on the detection signal S_{PSP}.

The control circuitry 119 further comprises a second electric switch 135 which is controlled by the output signal S_{H2} provided by the second magnetic-field sensor 123. Also the second electric switch 135 may comprise, e.g., a MOSFET. The second electric switch 135 is configured to be in a closed condition when the output signal S_{H2} of the second magnetic-field sensor 123 indicates the closed condition of the upper housing portion 27. The second electric switch 135 is configured to be in an opened condition when the output signal S_{H2} of the second magnetic-field sensor 123 indicates the opened condition of the upper housing portion 27.

The first and second electric switches 133, 135 are mutually connected in series and are connected in series with the rechargeable battery 109 of the auxiliary device 5, a boost convertor 137 of the control circuitry 119 and with each of the light sources 73a, 73b, 73c of the auxiliary device 5, wherein the light sources 73a, 73b, 73c may be mutually connected in parallel as shown in Fig. 12. Thus, to activate the light sources 73a, 73b, 73c, the first and second electric switches 133, 135 should be both in their closed condition.

When, in the opened condition of the upper housing portion 27, the user places the personal care device 3 in the predefined stored position within the lower housing portion 25, the detection signal S_{PSP} will indicate the presence of the personal care device 3 in the predefined stored position. Thereafter, as long as the upper housing portion 27 is not pivoted into its closed condition, the output signals S_{H1}, S_{H2} of the first and second magnetic-field sensors 121, 123 will not indicate the closed condition of the upper housing portion 27. Consequently, the first electric switch 133 will be remained in its opened condition by the processor 131 based on the output signal S_{H1}, and the second electric switch 135 will be remained in its opened condition directly based on the output signal S_{H2}. This will prevent the activation of the light sources 73a, 73b, 73c as long as the upper housing portion 27 is in its opened condition, more particularly as long as the upper housing portion 27 is not in its closed condition. This will prevent or at least reduce safety risks for the user, in particular the user's eyes, potentially caused by exposure to the disinfecting light when the upper housing portion 27 is not in its closed condition.

When the user, after placing the personal care device 3 in the predefined stored position, pivots the upper housing portion 27 into its closed condition, the output signals S_{H1}, S_{H2} of the first and second magnetic-field sensors 121, 123 will both indicate the closed condition of the upper housing portion 27. As a result, the second electric switch 135 will be closed directly based on the output signal S_{H2} and will remain closed as long as the output signal S_{H2} indicates the closed condition of the upper housing portion 27. Simultaneously, the processor 131 will close the first electric switch 133 based on the output signal S_{H1} and the detection signal S_{PSP} that indicates the presence of the personal care device 3 in the predefined stored position. The processor 131 will remain the first electric switch 133 closed during said predetermined time period, provided that the output signal S_{H1} remains indicating the closed condition of the upper housing portion 27. After said predetermined time period, the processor 131 opens the first electric switch 133, even when the output signal S_{H1} still indicates the closed condition of the upper housing portion 27. Thus, after the user pivots the upper housing portion 27 into its closed condition, the processor 131 will automatically start the disinfecting process by closing the first electric switch 133 and, thereby, activating the light sources 73a, 73b, 73c. The processor 131 will also automatically terminate the disinfecting process after said predetermined time period by opening the first electric switch 133 and, thereby, deactivating the light sources 73a, 73b, 73c. The predetermined time period may be, e.g., 10 minutes or any other time period sufficient to effectively and efficiently disinfect the personal care unit 9 of the personal care device 3. The progress of the disinfecting process may be indicated to the user by means of a second light indicator 139 which is arranged on the top side 51 of the upper housing portion 27, as shown in Figs. 4 and 10. The second light indicator 139 may, e.g., generate blinking light (e.g. blue light) as long as the light sources 73a, 73b, 73c are active, and may, e.g., continuously generate light or be deactivated after the automatic termination of the disinfecting process by the processor 131 upon expiry of the predetermined time period.

When the user pivots the upper housing portion 27 into its closed condition without first placing the personal care device 3 in the predefined stored position, the processor 131 will not detect the presence of the personal care device 3 in the auxiliary device 5 based on the detection signal S_{PSP} and, consequently, will not start the disinfecting process by activating the light sources 73a, 73, 73c. This will prevent any unnecessary activation of the light sources 73a, 73b, 73c and energy consumption.

When during the disinfecting process, i.e., during said predetermined time period, the user opens the auxiliary device 5 either deliberately or unintentionally by moving the upper housing portion 27 into its opened condition, the output signal S_{H1} of the first magnetic-field sensor 121 will no longer indicate the closed condition of the upper housing portion 27. Consequently, the processor 131 will open the first electric switch 133 and, thereby, automatically deactivate the light sources 73a, 73b, 73c as a safety measure. Simultaneously, also the output signal S_{H2} of the second magnetic-field sensor 123 will no longer indicate the closed condition of the upper housing portion 27, so that also the second electric switch 135 will be opened directly based on the output signal S_{H2}. In this situation, the second light indicator 139 may indicate the interruption of the disinfecting process by means of a dedicated light output. When, subsequently, the user closes the auxiliary device 5 again, the second electric switch 135 may be closed again based on the output signal S_{H2}, and the processor 131 may close the first electric switch 133 again based on the output signal S_{H1} and may remain the first electric switch 133 closed for a remaining part of the predetermined time period as long as the output signal S_{H1} indicates the closed condition of the upper housing portion 27.

The detection signal S_{PSP} received from the electric charging contact 63 of the auxiliary device 5, as described herein before, and the output signal S_{H1} of the first magnetic-field sensor 121 may also be used as input signals for the electrical power circuitry of the main PCB 111 to control the first light indicator 115 indicating the charging level of the rechargeable battery 109 of the auxiliary device 5. In particular, the electrical power circuitry may activate the first light indicator 115 only for a limited time period, e.g., a few seconds, upon detection of placement of the auxiliary device 5 in the predefined stored position based on the detection signal S_{PSP}, and/or upon detection of pivoting of the upper housing portion 27 out of its closed condition based on the output signal S_{H1}, and/or upon detection of removal of the auxiliary device 5 from the predefined stored position based on the detection signal S_{PSP}.

It will be evident for the skilled person that the above-described safety measure of automatically preventing and interrupting the activation of the light sources 73a, 73b, 73c by means of the control circuitry 119 will also work in the absence of the second magnetic-field sensor 123 and the second electric switch 135. The second magnetic-field sensor 123 and the second electric switch 135 may therefore be regarded as constituting an additional safety measure. It will further be evident for the skilled person that the first and second magnetic-field sensors 121, 123 may be arranged on the upper housing portion 27 and the first and second magnets 125, 127 may be arranged on the lower housing portion 25. The arrangement of the first and second magnetic-field sensors 121, 123 on the lower housing portion 25 is however preferred in view of the required electric connection of the first and second magnetic-field sensors 121, 123 with the processor 131 of the control circuitry 119 when being part of the main PCB 111 in the lower housing portion 25.

As further shown in Fig. 4, in the embodiment of the personal care system 1 the lower housing portion 25 of the auxiliary device 5 comprises a third magnet 141 and a fourth magnet 143. The third magnet 141 is arranged to be magnetically attracted by the first magnet 125 in the closed condition of the upper housing portion 27. Similarly, the fourth magnet 143 is arranged to be magnetically attracted by the second magnet 127 in the closed condition of the upper housing portion 27. For this purpose and as shown in Fig. 4, the third magnet 141 and the fourth magnet 143 are arranged in positions close to, respectively, the first magnetic-field sensor 121 and the second magnetic-field sensor 123, i.e., in two respective positions close to and mutually opposite relative to the lowest position P_{LOW} on the edge structure 89 of the lower housing portion 25. The magnetic attraction of the first and third magnets 125, 141 and the magnetic attraction of the second and fourth magnets 127, 143 provide a solid closing force maintaining the upper housing portion 27 in its closed condition relative to the lower housing portion 25. In particular, the first, second, third and fourth magnets 125, 127, 141, 143 may be configured and arranged to provide a closing force which is sufficiently high to enable the user to carry and hold the auxiliary device 5 at the upper housing portion 27 in its closed condition without the risk that the upper housing portion 27 will pivot out of its closed condition.

As shown in Figs. 1, 2, 3 and 9, in the embodiment of the personal care system 1 the personal care device 3 comprises a third light indicator 145. The third light indicator 145 is provided on the second portion 65 of the main body 7 of the personal care device 3, in particular near the second end portion 17 of the main body 7. In the embodiment shown, the third light indicator 145 has an annular shape and, thus, extends as a closed loop circumferentially about the second portion 65 of the main body 7. The third light indicator 145 is configured to emit, during use of the personal care device 3, i.e. the electric shaver, light of different colors in dependence on a pressure which is exerted on the personal care unit 9, i.e., the shaving unit, and which is measured by means of a pressure sensor in the personal care device 3. Such a light indicator to provide the user with feedback about the applied pressure is disclosed by EP3849759B1 and EP3856473B1 in the name of the applicant. As shown in Fig. 3, in the embodiment of the personal care system 1 the personal care device 3 further comprises a display 147. The display 147 is provided on the first portion 55 of the main body 7 of the personal care device 3. The display 147 may be used to display, e.g., particular information relating to the operation of the personal care device 3 during use thereof, as is well known to the skilled person.

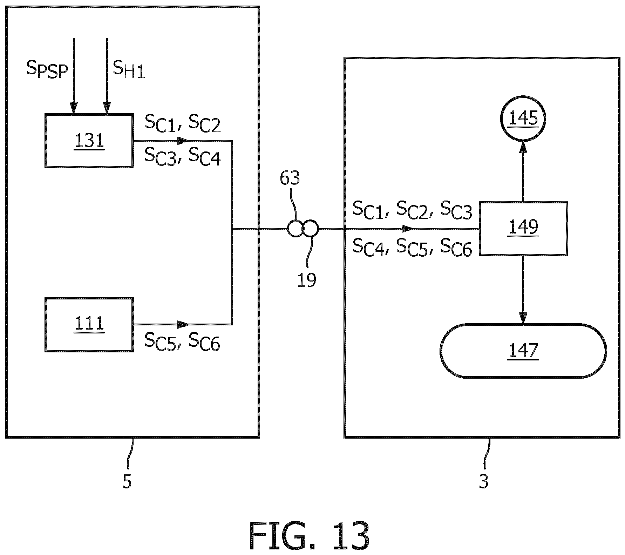

In view of its arrangement on the second portion 65 of the main body 7, which protrudes from the lower housing portion 25 of the main body 7 via the receiving opening 37 when the personal care device 3 is in its predefined stored position within the lower housing portion 25, the third light indicator 145 of the personal care device 3 remains visible to the user in the opened condition of the upper housing portion 27 and in the predefined stored position of the personal care device 3, as is shown in Figs. 1 and 2. In view of its arrangement on the first portion 55 of the main body 7, which is received by the first receiving chamber 35 of the lower housing portion 25 when the personal care device 3 is in its predefined stored position within the lower housing portion 25, the display 147 is however not visible to the user in the predefined stored position of the personal care device 3. Fig. 13 schematically shows a control system of the personal care system 1 for controlling the third light indicator 145 and the display 147 of the personal care device 3 when interacting with the auxiliary device 5.

As shown in Fig. 13, the personal care device 3 comprises a processor 149. The processor 149 of the personal care device 3 may control several electrical functions of the personal care device 3, as is well known to the skilled person. The processor 149 of the personal care device 3 is configured to receive electric communication signals Sci, S_{C2}, S_{C3}, S_{C4}, Scs, S_{C6} from the auxiliary device 5 via the electric charging contacts 19, 63 of the personal care device 3 and the auxiliary device 5 when they mutually engage in the predefined stored position of the personal care device 3 within the lower housing portion 25 of the auxiliary device 5.

As shown in Fig. 13, the processor 149 of the personal care device 3 may in particular receive a first predefined communication signal Sci, a second predefined communication signal S_{C2}, a third predefined communication signal Scs and a fourth communication signal S_{C4} which are each transmitted by the processor 131 of the control circuitry 119 of the auxiliary device 5. The processor 131 of the auxiliary device 5 is configured to transmit the first predefined communication signal Sci when the electric detection signal S_{PSP}, received by the processor 131 of the auxiliary device 5 as described herein before, indicates placement of the personal care device 3 in the predefined stored position. The processor 131 of the auxiliary device 5 is configured to transmit the second predefined communication signal S_{C2} when the electric detection signal S_{PSP}, received by the processor 131 of the auxiliary device 5, indicates removal of the personal care device 3 from the predefined stored position. The processor 131 of the auxiliary device 5 is configured to transmit the third predefined communication signal S_{C3} when the output signal S_{H1} of the first magnetic-field sensor 121, received by the processor 131 of the auxiliary device 5 as described herein before, indicates termination of the closed condition of the upper housing portion 27 of the auxiliary device 5. The fourth communication signal S_{C4} represents a status of the disinfecting process of the personal care unit 9 of the personal care device 3 by means of the auxiliary device 5. The fourth communication signal S_{C4} may, e.g., indicate that the disinfecting process is completed and terminated, i.e., that the light sources 73a, 73b, 73c have been deactivated after said predetermined time period.

As shown in Fig. 13, the processor 149 of the personal care device 3 may further receive a fifth communication signal Scs and a sixth communication signal S_{C6} which are transmitted by the electrical power circuitry of the main PCB 111 of the auxiliary device 5. The fifth communication signal Scs represents a status of the charging process of the battery 11 of the personal care device 3 by means of the electrical power circuitry of the auxiliary device 5. The fifth communication signal Scs may indicate, e.g., the charging level of the battery 11 of the personal care device 3 or the completion of the charging process of the battery 11 of the personal care device 3 by means of the auxiliary device 5. The sixth communication signal S_{C6} represents a status of the rechargeable battery 109 of the auxiliary device 5, in particular a status of the charging process of the rechargeable battery 109 by means of the electrical power circuitry of the auxiliary device 5. The sixth communication signal S_{C6} may indicate, e.g., the charging level of the rechargeable battery 109 of the auxiliary device 5.

The processor 149 of the personal care device 3 is configured to control the third light indicator 145 of the personal care device 3 to generate a first predefined light output in reply to receipt by the processor 149 of the first predefined communication signal Sci. The first predefined light output may, e.g., be a short light flash of a particular color. This will indicate to the user the correct placement of the personal care device 3 in the predefined stored position within the lower housing portion 25, i.e., when the user places the personal care device 3 into the auxiliary device 5 in the opened condition of the upper housing portion 27.

The processor 149 of the personal care device 3 is further configured to deactivate the display 147 in reply to receipt by the processor 149 of the first predefined communication signal S_{C1}. Thus, the display 147 is automatically deactivated after placement of the personal care device 3 in the predefined stored position within the lower housing portion 25, i.e., when the display 147 is no longer visible for the user. This will save energy consumption of the personal care system 1 and may extend the lifetime of the display 147.

The processor 149 of the personal care device 3 is further configured to control the third light indicator 145 of the personal care device 3 to generate a second predefined light output in reply to receipt by the processor 149 of the third predefined communication signal S_{C3}. The second predefined light output may be generated only when, in addition, the fourth communication signal S_{C4} received by the processor 149 indicates that the disinfecting process is completed and terminated. In this way, when the user opens the auxiliary device 5 by pivoting the upper housing portion 27 into its opened condition, the third light indicator 145 of the personal care device 3 may indicate to the user, e.g., by a short light flash of a dedicated color, that the disinfecting process was completed. Alternatively, the second predefined light output may be generated in dependence on the fourth communication signal S_{C4} received by the processor 149. In this way, when the auxiliary device 5 is opened, the third light indicator 145 of the personal care device 3 may indicate, e.g., by means of light flashes of different colors, whether or not the disinfecting process was completed.

The processor 149 of the personal care device 3 is further configured to control the display 147 of the personal care device 3 in reply to receipt by the processor 149 of the second predefined communication signal S_{C2}. In particular, upon receipt of the second predefined communication signal S_{C2} the processor 149 of the personal care device 3 controls the display 147 to display information relating to the status of the disinfecting process of the personal care unit 9 of the personal care device 3 by means of the auxiliary device 5, represented by the fourth communication signal S_{C4} received by the processor 149, information relating to the status of the charging process of the battery 11 of the personal care device 3 by means of the auxiliary device 5, represented by the fifth communication signal Scs received by the processor 149, and information relating to the status of the charging process of the rechargeable battery 109 of the auxiliary device 5, represented by the sixth communication signal S_{C6} received by the processor 149. In this way, when the user removes the personal care device 3 from the auxiliary device 5 and the display 147 of the personal care device 3 becomes visible again to the user, the display 147 may display, e.g., by means of dedicated symbols, that the disinfecting process by means of the auxiliary device 5 was completed, the charging level of the battery 11 of the personal care device 3, and the charging level of the rechargeable battery 109 of the auxiliary device 5. The display 147 may display these different information types simultaneously, or the user may cause the display 147 to sequentially display these different information types by means of a suitable user interface provided on the personal care device 3.

Variations to the disclosed embodiments can be understood and effected by those skilled in the art in practicing the principles and techniques described herein, from a study of the figures, the disclosure and the appended claims. In the claims, the word "comprising" does not exclude other elements or steps, and the indefinite article "a" or "an" does not exclude a plurality. The mere fact that certain measures are recited in mutually different dependent claims does not indicate that a combination of these measures cannot be used to advantage. Any reference numbers in the claims should not be construed as limiting the scope of the claims.

## Claims

1. An auxiliary device (5) for use with an electric personal care device (3) and configured to store the personal care device, to electrically charge a battery (11) of the personal care device, and to disinfect a personal care unit (9) of the personal care device by means of disinfecting light, wherein:
- the personal care device comprises a main body (7) accommodating the battery and having a longitudinal axis (13) which extends from a first end portion (15) of the main body to a second end portion (17) of the main body;
- the first end portion is provided with an electric charging contact (19) for charging the battery;
- the personal care unit is coupled to the second end portion;
- the main body has a first portion (55) which includes the first end portion, extends along the longitudinal axis from the first end portion towards the second end portion, and excludes a second portion (65) of the main body, said second portion including the second end portion and extending along the longitudinal axis from the second end portion towards the first end portion; and
- the main body and the second portion of the main body have, respectively, a first average length (L₁) and a second average length (L₂) measured parallel to the longitudinal axis;
wherein the auxiliary device comprises a housing (23) having a lower housing portion (25) and an upper housing portion (27), wherein the lower housing portion comprises:
- a bottom side (33) configured to be supportable in a horizontal orientation which defines a normal operational position of the auxiliary device;
- an upper side (39) provided with a receiving opening (37);
- a longitudinal axis (41) extending, in the normal operational position, in a vertical direction from the bottom side to the upper side; and
- a first receiving chamber (35) which is accessible via the receiving opening and configured to receive the first portion of the main body in a predefined stored position of the personal care device;
wherein the upper housing portion:
- has a closed condition and an opened condition relative to the lower housing portion; and
- comprises a second receiving chamber (43) which is configured to receive, in the closed condition of the upper housing portion and in the predefined stored position of the personal care device, the second portion of the main body and the personal care unit coupled to the main body;
and wherein:
- the lower housing portion and the upper housing portion are configured to fully enclose the main body and the personal care unit coupled to the main body in the closed condition of the upper housing portion and in the predefined stored position of the personal care device;
- the lower housing portion is configured such that, in the opened condition of the upper housing portion and in the predefined stored position of the personal care device, the second portion of the main body protrudes from the lower housing portion via the receiving opening;
- the lower housing portion comprises an electric charging contact (63) arranged to engage the electric charging contact of the main body in the predefined stored position of the personal care device;
- the upper housing portion comprises at least one light source (73a, 73b, 73c) configured to generate the disinfecting light and arranged to expose the personal care unit, when coupled to the main body, with the disinfecting light in the closed condition of the upper housing portion and in the predefined stored position of the personal care device; and
- a ratio (L₂/L₁) between the second average length and the first average length is at least 0.05.

2. The auxiliary device (5) as claimed in claim 1, wherein the ratio (L₂/L₁3. between the second average length (L₂) and the first average length (L₁) is from 0.1 to 0.35.

3. The auxiliary device (5) as claimed in claim 1 or 2, wherein the upper housing portion (27) is pivotable relative to the lower housing portion (25) from the closed condition to the opened condition, and vice versa, by means of a hinge structure (29).

4. The auxiliary device (5) as claimed in claim 3, wherein:
- a main orientation of the receiving opening (37) of the lower housing portion (25) is oblique relative to the longitudinal axis (41) of the lower housing portion;
- the lower housing portion has an edge structure (89) surrounding the receiving opening; and
- the hinge structure (29) is provided in a position (P_{HS}) on the edge structure where the edge structure has a largest distance (D_{MAX}) from the bottom side (33) of the lower housing portion, measured in a direction parallel to the longitudinal axis of the lower housing portion.

5. The auxiliary device (5) as claimed in any of claims 1-3, wherein:
- an upper side (93) of the main body (7), which is provided at the second end portion (17) of the main body, has a main orientation which is oblique relative to the longitudinal axis (13) of the main body; and
- a main orientation of the receiving opening (37) of the lower housing portion (25) is parallel to the main orientation of the upper side of the main body in the predefined stored position of the personal care device (3).

6. The auxiliary device (5) as claimed in any of claims 1-5, wherein:
- the electric charging contact (19) of the personal care device (3) is provided on a lower side (57) of the main body (7) provided at the first end portion (15) of the main body;
- the electric charging contact (63) of the auxiliary device is provided on a bottom surface (61) of the first receiving chamber (35); and
- the first receiving chamber tapers in a direction along the longitudinal axis (41) of the lower housing portion (25) from the receiving opening (37) towards the bottom surface.

7. The auxiliary device (5) as claimed in any of claims 1-6, wherein:
- the personal care device (3) is an electric shaver and the personal care unit (9) is a shaving unit comprising three circularly shaped hair-cutting units (21a, 21b, 21c) which are arranged triangularly on a supporting member (22) of the shaving unit; and
- the upper housing portion (27) comprises three light sources (73a, 73b, 73c), in particular three UV-LEDs, arranged triangularly to each mainly expose a respective one of the three hair-cutting units of the shaving unit, when coupled to the main body (7), with the disinfecting light in the closed condition of the upper housing portion and in the predefined stored position of the electric shaver.

8. The auxiliary device (5) as claimed in any of claims 1-7, wherein:
- one of the lower housing portion (25) and the upper housing portion (27) comprises a first magnet (125), and the other one of the lower housing portion and the upper housing portion comprises a first magnetic-field sensor (121), in particular a first Hall sensor;
- the first magnet and the first magnetic-field sensor are arranged to enable the first magnetic-field sensor to detect different magnetic-field strengths in, respectively, the closed condition and the opened condition of the upper housing portion; and
- the auxiliary device comprises a processor (131) configured to activate the at least one light source (73a, 73b, 73c) for a predetermined time period upon detection of both the closed condition of the upper housing portion by means of the first magnetic-field sensor and the predefined stored position of the personal care device (3) by means of an electric detection signal (S_{PSP}) exchanged via the electric charging contacts (19, 63) of the auxiliary device and the personal care device when mutually engaging.

9. The auxiliary device (5) as claimed in claim 8, wherein the processor (131) is configured to deactivate the at least one light source (73a, 73b, 73c) and/or disable activation of the at least one light source when the first magnetic-field sensor (121) does not detect the closed condition of the upper housing portion (27).

10. The auxiliary device (5) as claimed in claim 8 or 9, wherein:
- the processor (131) is configured to activate the at least one light source (73a, 73b, 73c) by closing a first electric switch (133) connected in series with the at least one light source;
- said one of the lower housing portion (25) and the upper housing portion (27) comprises a second magnet (127) and said other one of the lower housing portion and the upper housing portion comprises a second magnetic-field sensor (123), in particular a second Hall sensor;
- the second magnet and the second magnetic-field sensor are arranged to enable the second magnetic-field sensor to detect different magnetic-field strengths in, respectively, the closed condition and the opened condition of the upper housing portion; and
- the auxiliary device comprises a second electric switch (135) connected in series with the first electric switch and the at least one light source and configured to be opened as a result of an output signal (S_{H2}) provided by the second magnetic-field sensor when the second magnetic-field sensor does not detect the closed condition of the upper housing portion.

11. The auxiliary device (5) as claimed in any of claims 8-10, wherein said other one of the lower housing portion (25) and the upper housing portion (27) comprises a third magnet (141) arranged to be magnetically attracted by the first magnet (125) in the closed condition of the upper housing portion.

12. The auxiliary device (5) as claimed in any of claims 1-11, wherein the auxiliary device comprises a rechargeable battery (109) configured and arranged to power the at least one light source (73a, 73b, 73c) and to charge the battery (11) of the personal care device (3) in the predefined stored position of the personal care device.

13. A personal care system (1) comprising an electric personal care device (3) and an auxiliary device (5) which is configured to store the personal care device, to electrically charge a battery (11) of the personal care device, and to disinfect a personal care unit (9) of the personal care device by means of disinfecting light, wherein:
- the personal care device comprises a main body (7) accommodating the battery and having a longitudinal axis (13) which extends from a first end portion (15) of the main body to a second end portion (17) of the main body;
- the first end portion is provided with an electric charging contact (19) for charging the battery;
- the personal care unit is coupled to the second end portion;
- the main body has a first portion (55) which includes the first end portion, extends along the longitudinal axis from the first end portion towards the second end portion, and excludes a second portion (65) of the main body, said second portion including the second end portion and extending along the longitudinal axis from the second end portion towards the first end portion; and
- the main body and the second portion of the main body have, respectively, a first average length (L₁) and a second average length (L₂) measured parallel to the longitudinal axis;
and wherein the auxiliary device is an auxiliary device as claimed in any of claims 1-12.

14. The personal care system (1) as claimed in claim 13, wherein:
- the second portion (65) of the main body (7) of the personal care device (3) comprises a light indicator (145);
- the personal care device comprises a processor (149) configured to receive an electric communication signal (Sci, Scs) from a processor (131) of the auxiliary device (5) via the electric charging contacts (19, 63) of the personal care device and the auxiliary device when mutually engaging;
- the processor of the auxiliary device is configured to transmit, via said electric charging contacts when mutually engaging, a predefined communication signal (Sci, Scs) to the processor of the personal care device in reply to at least one of the following: placement of the personal care device in the predefined stored position; termination of the closed condition of the upper housing portion (27) of the auxiliary device detected by a sensor (121) of the auxiliary device; and
- the processor of the personal care device is configured to control the light indicator to provide a predefined light output in reply to receipt of said predefined communication signal.

15. The personal care system (1) as claimed in claim 13, wherein:
- the first portion (55) of the main body (7) of the personal care device (3) comprises a display (147);
- the personal care device comprises a processor (149) configured to receive an electric communication signal (S_{C2}, S_{C4}, Scs) from a processor (111, 131) of the auxiliary device (5) via the electric charging contacts (19, 63) of the personal care device and the auxiliary device when mutually engaging;
- the processor of the auxiliary device is configured to transmit, via said electric charging contacts when mutually engaging, a communication signal (S_{C4}, Scs) representing a status of charging the battery (11) of the personal care device and/or disinfecting the personal care unit (9) of the personal care device by means of the auxiliary device; and
- the processor of the personal care device is configured to control the display to display information relating to said status of charging and/or disinfecting, represented by the transmitted communication signal (S_{C4}, Scs), upon indication by the transmitted communication signal (S_{C2}) of removal of the personal care device from the predefined stored position.

16. The personal care system (1) as claimed in claim 15, wherein:
- the auxiliary device (5) is an auxiliary device as claimed in claim 12;
- the transmitted communication signal (S_{C6}) further represents a status of the rechargeable battery (109) of the auxiliary device; and
- the processor (149) of the personal care device (3) is configured to control the display (147) to further display information relating to said status of the rechargeable battery of the auxiliary device, represented by the transmitted communication signal (S_{C6}), upon indication by the transmitted communication signal (S_{C2}) of removal of the personal care device from the predefined stored position.

17. The personal care system (1) as claimed in claim 15 or 16, wherein:
- the processor (131) of the auxiliary device (5) is configured to transmit, via said electric charging contacts (19, 63) when mutually engaging, a predefined communication signal (Sci) to the processor (149) of the personal care device (3) in reply to placement of the personal care device in the predefined stored position; and
- the processor of the personal care device is configured to deactivate the display (147) in reply to receipt of said predefined communication signal.
